# EUROPÄISCHE PATENTANMELDUNG

(11) **EP 2 767 821 A1**
(43) Veröffentlichungstag der Anmeldung: **20.08.2014**
(21) Anmeldenummer: 14154282.9
(22) Anmeldetag: 07.02.2014
(51) Int. Cl.: G01N 21/27, G01N 21/33

(54) **Fotometrische Messvorrichtung und Verfahren zum Messen einer optischen Absorbanz eines Fluids mit einer variablen Konzentration von mindestens einer Licht absorbierenden Substanz, sowie Blutbehandlungsvorrichtung mit einer derartigen Messvorrichtung**

(30) Priorität: 15.02.2013 DE 102013101523
(71) Anmelder: B. Braun Avitum AG, 34212 Meisungen (DE)
(72) Erfinder: Ahrens, Jörn, 34225 Baunatal (DE); Moll, Stefan, 22587 Hamburg (DE); Castellarnau, Alex, 215021 Suzhou (CN)
(74) Vertreter: Winter, Brandl, Fürniss, Hübner, Röss, Kaiser, Polte - Partnerschaft mbB

(57) **Zusammenfassung**

Offenbart werden Messvorrichtungen und Verfahren zum Messen einer optischen Absorbanz eines in einem Fluidbehälter enthaltenen oder in einer Fluidleitung strömenden Fluids, wie etwa eines in einem Dialysatabfluss einer Dialysemaschine strömenden Dialysats. Gemäß einem ersten Aspekt umfasst der Fluidbehälter oder die Fluidleitung mindestens eine erste und eine zweite durch das Fluid verlaufende optische Messstrecke mit jeweils einer entsprechenden vordefinierbaren ersten und zweiten Länge, wobei die erste Länge größer als die zweite Länge ist. Die erste, längere optische Messstrecke dient zum Messen der Absorbanz mit hoher Auflösung in einem ersten Messkanal für den Fall, dass das Fluid eine erste z.B. relativ geringe bis keine Konzentration der Substanz enthält, während die zweite, kürzere optische Messstrecke zum Messen der Absorbanz mit hoher Auflösung dient. Gemäß einem zweiten Aspekt umfasst der Fluidbehälter oder die Fluidleitung mindestens eine durch das Fluid verlaufende optische Messstrecke mit einstellbarer Länge.

## Beschreibung

Die Erfindung betrifft eine fotometrische Messvorrichtung und ein Verfahren zum Messen einer optischen Absorbanz eines Fluids mit einer variablen Konzentration von mindestens einer Licht absorbierenden Substanz, sowie eine Blutbehandlungsvorrichtung, wie etwa eine Dialysemaschine, mit einer derartigen Messvorrichtung.

### Stand der Technik

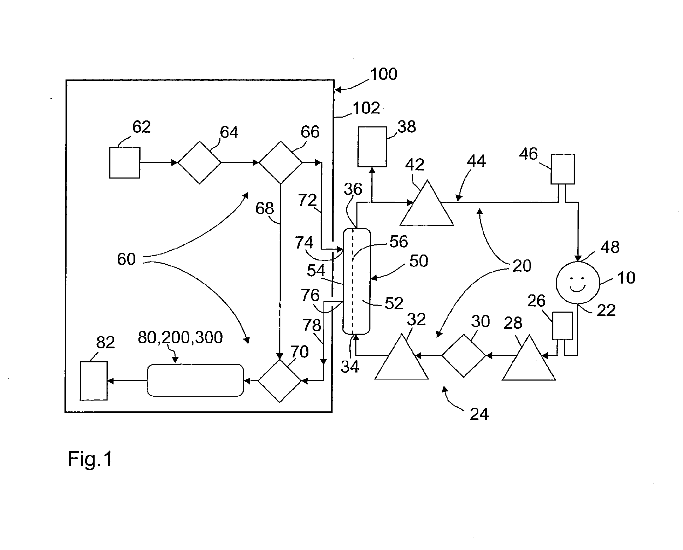

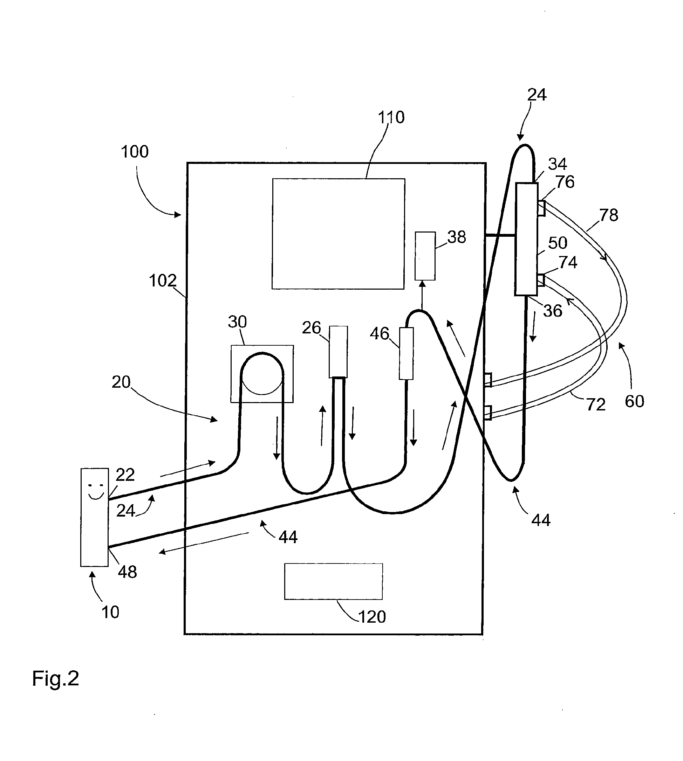

In jüngster Zeit hat die Anmelderin modularen Blutbehandlungsvorrichtungen, wie etwa die in den Figuren 1 und 2 schematisch gezeigten Dialysemaschinen 100, zum Behandeln von Blut eines Patienten 10, z. B. durch Stoffaustausch, in einem extrakorporalen Blutkreislauf 20, wobei der Stoffaustausch von im Blut enthaltenen Substanzen, wie etwa urämischen Toxinen, in einer Dialysatoreinrichtung 50 durch eine Membran eine in der Dialysatoreinrichtung 50 angeordneten Membranfiltereinrichtung 56 durchgeführt wird, und wobei die aus dem Blut des Patienten 10 zu entfernenden Substanzen durch die Membran in eine in einem Dialysatkreislauf 60 geführte Dialysierflüssigkeit bzw. Dialysat aufgenommen und in dem Dialysat gelöst in einen Dialysatabfluss 82 abgeführt werden. Dabei wird die Konzentration der von dem Dialysat durch die Membran aus dem Blut des Patienten 10 aufgenommenen Substanzen in einer Dialysatabfuhrleitung 78 einer Konzentrationsmesseinrichtung 80 zum Messen der Konzentration/en von einer oder mehreren der im Dialysat aufgenommenen Substanzen zugeführt.

Früher wurde zum Bestimmen der Konzentrationen der Substanzen im Dialysat elektrische Leitfähigkeitsmessmethoden eingesetzt, wie beispielsweise in DE 32 23 051 A1 und in EP 0 428 927 A1 beschrieben. In jüngster Zeit wurden auf UV-spektrophotometrischen Methoden basierende Vorrichtungen und Verfahren zur Bestimmung der Konzentrationen von aus dem Blut entzogenen Substanzen, wie etwa urämischen Toxinen untersucht, wie etwa in den Arbeiten von Fridolin et al. (Uhlin F., Haemodiaysis Treatment monitored online by Ultraviolet Absorbance, Linköping University Medical Dissertations No. 962, Department of Medicine and Care, Division of Nursing Science and Department of Biomedical Engineering) 2006 erforscht und in EP 1 083 948 B1 beschrieben. Dabei ist die Konzentrationsmesseinrichtung 80 als im ultravioletten (UV) Spektralbereich arbeitende spektralphotometrische Messeinrichtung realisiert, die auf bekannten, substanzspezifischen und wellenlängenselektiven Messungen der optischen Absorbanzen von aus dem Blut entzogenen und in der Dialysatabfuhr im Dialysat mitgeführten und zum Teil gelösten Substanzen beruht. Je nach dem substanzspezifischen Absorptionsspektrum einer Substanz wird Licht mit verschiedenen (UV) Wellenlängen in unterschiedlichen Maßen beim Durchlaufen einer durch das Dialysat führenden optischen Messstrecke absorbiert.

Dazu wird in US 2012/0154789 A1 eine optische (z. B. im UV funktionierende) Sensoreinrichtung zum optischen Überwachen eines Fluids, wie etwa Blut, das durch eine Messkammer strömt, vorgeschlagen, die bei entsprechender Ausgestaltung der optischen Messkammer auch zum optischen Überwachen von Konzentrationen von mitgeführten bzw. gelösten Substanzen im Dialysat eingesetzt werden kann. Diese Sensorvorrichtung umfasst eine als (UV) LED ausgebildete Lichtquelle, die Licht durch die von dem Fluid durchströmte Messkammer entlang einer durch die Messkammer definierten optischen Messstrecke ausstrahlt, und beispielsweise als lichtempfindliche Dioden ausgebildete Lichtdetektoren, die zum Messen der durch die Messkammer hindurchtretenden (durch Absorption bzw. Lichtstreuung geschwächten) Lichtintensität ausgebildet und auf der im Lichtdetektor gegenüberliegenden Seite der Messkammer angeordnet sind. Dabei wird davon ausgegangen, dass eine derartige kompakte Lichtquelle Licht bei einer wohldefinierten Wellenlänge bzw. in einem engen spektralen Wellenlängenband abstrahlt. Auch in den von der Anmelderin entwickelten Blutbehandlungsvorrichtungen 100 wird in der UV-Messeinrichtung 80 eine derartige engwandige UV-LED eingesetzt, die ein relativ schmales Emissionsband mit einer nominalen Wellenlänge (Peak-Wellenlänge) bei 280 nm emittiert.

Die vorliegende Erfindung beruht nun zum einen auf dem Wunsch, das bekannte UV-Absorptionsmessverfahren dahingehend weiterzuentwickeln und zu verbessern, dass Konzentrationen von bestimmten Substanzen mit hoher Auflösung (bezüglich der Konzentration bzw. der optischen Absorbanz) sowohl bei relativ geringen bis verschwindenden Konzentrationen der Substanzen, die eine entsprechende relativ geringe Absorption bzw. eine relativ hohe durch die optische Messstrecke hindurchtretende Lichtintensität verursachen, als auch relativ hohe Konzentrationen der Substanzen erfasst werden können, die entsprechend eine relativ hohe Absorption bzw. Streuung und eine relativ niedrige durch die optische Messstrecke hindurchtretende Lichtintensität bewirken.

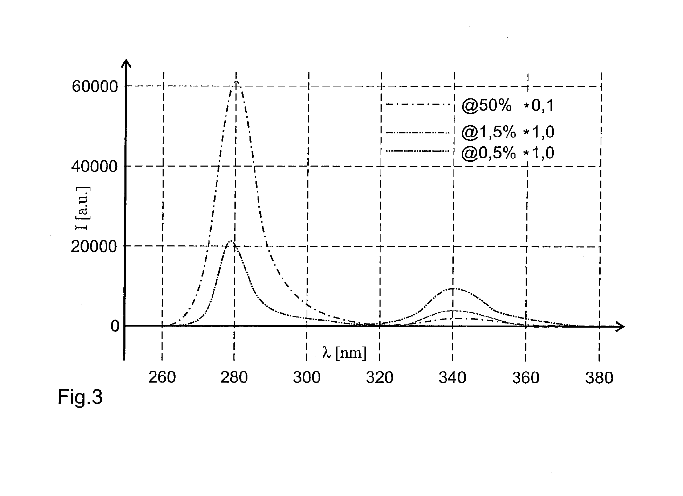

Überdies hat die Erfinder festgestellt, dass die in den gängigen UV-Messeinrichtungen typischerweise verwendeten, kommerziell erhältlichen UV-LEDs mit gewünschtem Emissionsband bei 280 nm eine wohl durch Verunreinigungen im Halbleitermaterial der LED verursachte Strahlung mit einer relativ geringen Intensität in einem Emissionsband, das bei einer nicht erwünschten Wellenlänge, nämlich ca. 340 nm, wie in Fig. 3 veranschaulicht, emittiert. Insbesondere wurde festgestellt, dass die Intensität dieser unerwünschten Lichtemission bei der nicht erwünschten Wellenlänge nicht proportional zum Steuerstrom der LED ist, mit dem die Intensität des gewünschten Emissionsbands bei der gewünschten Emissionswellenlänge gesteuert wird, sondern sich im Wesentlichen besonders bei sehr geringen Steuerströmen (entsprechend geringen gewünschten Lichtemissionen) ausbildet. Die Verhältnisse sind in der Fig. 3 veranschaulicht. Das in Fig. 3 mit den Symbolen "...." gezeigte Emissionsspektrum wurde bei einem Steuerstrom in Höhe von 50 % der maximalen LED-Lichtausgangsleistung aufgenommen und die gemessenen Intensitätswerte für Darstellungszwecke mit einem Faktor 0,1 (10 %) multipliziert dargestellt. Die in Fig. 3 mit den Symbolen "□□□□" gekennzeichnete Kurve wurde mit einem Steuerstrom für 1,5 % der maximalen Lichtleistung der LED aufgenommen, und man sieht, dass sowohl die gewünschte Emissionsbande (bei 280 nm) als auch die unerwünschte Emissionsbande (bei 340 nm) noch nahezu proportional zueinander sich ausbilden. Das in Fig. 3 mit den Symbolen "••••" gekennzeichnete Emissionsspektrum wurde bei einem noch geringeren Steuerstrom entsprechend 0,5 % der maximalen Lichtleistung aufgenommen und zeigt sogar eine bei geringen Lichtintensitäten zunächst nur im unerwünschten Wellenlängenbereich ausgebildete Lichtemission der UV-LED. Angesichts des in Fig. 3 veranschaulichten Emissionsverhaltens der bisher eingesetzten Lichtquelle entstand die vorliegende Erfindung zum Weiteren mit dem Ziel, den Einfluss der nicht erwünschten Lichtemission in Bezug auf die gewünschte Lichtemission bei der Sollwellenlänge zu minimieren.

Der Erfindung liegt die Aufgrabe zugrunde, eine photometrische Messvorrichtung und ein Verfahren zum Messen einer optischen Absorbanz eines z.B. in einem Fluidbehälter enthaltenen oder in einer Fluidleitung strömenden Fluids, z. B. eines im Dialysatabfluss einer Dialysemaschine strömenden Dialysats, das aufgrund einer z. B. zeitlich variablen Zusammensetzung oder einer zeitlich variierbaren bzw. variablen Konzentration von einer oder mehreren in dem Fluid enthaltenen (z. B. gelösten oder mitgeführten) Substanzen eine (z. B. zeitlich) relativ stark variierende Absorbanz aufweisen kann, mit hoher photometrischer Genauigkeit und bei minimiertem Einfluss von unerwünschten Lichtemissionen der Lichtquelle bei nicht erwünschten Wellenlängen auf die gewünschte Lichtemission bei der gewünschten Wellenlänge, zu schaffen.

Ein erster Grundgedanke zur Lösung der Aufgabe besteht darin, eine durch das Fluid verlaufende erste und zweite optische Messstrecke mit einer vordefinierbaren ersten und zweiten Länge bereitzustellen, wobei die erste Länge größer als die zweite Länge ist und wobei die längere optische Messstrecke in einem ersten Kanal dazu dient, relativ geringe Konzentrationen der Substanzen in dem Fluid zu messen, während die zweite kürzere optische Messstrecke dazu dient, eine relativ hohe Konzentration der in dem Fluid enthaltenen Substanz zu messen und/oder einen redundanten zweiten Messkanal zur Verfügung zu stellen. In Abwandlung zum ersten Grundgedanken kann auch eine optische Messstrecke mit einstellbarer Länge bereitgestellt werden und damit Messungen bei einer ersten, z. B. größeren eingestellten Länge und bei einer zweiten, z. B. kürzeren eingestellten Länge durchzuführen.

Ein zweiter Grundgedanke zur Lösung der Aufgabe ist es, die Messvorrichtung bei einer, z. B. relativ geringen bis verschwindenden, Grundkonzentration der zu messenden Substanzen hinsichtlich der von der Lichtquelle emittierten Lichtleistung mit der gewünschten optischen Emissionscharakteristik so zu kalibrieren, dass ein erster Lichtintensitätsdetektor in einem Dynamikbereich mit darin enthaltenem Messbereich mit optimaler Auflösung und insbesondere optimaler Linearität zwischen dessen Ausgangssignal und der durch die erste optische Messstrecke durchtretende erste Lichtintensität betrieben wird und die ausgesendete Lichtintensität der Lichtquelle auf der Grundlage einer Referenzintensitätsmessung der von der ersten Lichtquelle ausgesendeten Lichtintensität auf ein zeitlich konstantes Niveau der ausgesendeten Lichtintensität geregelt wird.

Die oben genannte Aufgabe wird gelöst gemäß einem ersten Aspekt der Erfindung durch eine fotometrische Messvorrichtung mit den Merkmalen des unabhängigen Patentanspruches 1, gemäß einem zweiten Aspekt durch eine fotometrische Messvorrichtung mit den Merkmalen des unabhängigen Patentanspruches 7, gemäß einem dritten Aspekt durch ein Verfahren mit den Merkmalen des unabhängigen Patentanspruchs 13 und gemäß einem vierten Aspekt durch ein Verfahren mit den Merkmalen des unabhängigen Patentanspruchs 14. Vorteilhafte Weiterbildungen sind Gegenstände der abhängigen Patentansprüche.

Gemäß einem ersten Aspekt der Erfindung wird eine fotometrische Messvorrichtung bereitgestellt zum Messen einer optischen Absorbanz eines in einem Fluidbehälter enthaltenen oder in einer Fluidleitung strömenden Fluids, wie etwa eines in einem Dialysatabfluss einer Dialysemaschine strömenden Dialysats, wobei das Fluid entlang der optischen Messstrecke aufgrund einer zeitlich variablen Zusammensetzung oder einer zeitlich variierbaren bzw. variablen Konzentration von einer oder mehreren in dem Fluid enthaltenen, z.B. gelösten oder mitgeführten, Substanzen eine zeitlich variierende Absorbanz aufweisen kann.

Diese Messvorrichtung kann gemäß dem ersten Aspekt eines, mehrere oder alle der folgenden Merkmale umfassen:
den Fluidbehälter oder die Fluidleitung, der oder die mindestens eine erste und eine zweite durch das Fluid verlaufende optische Messstrecke mit jeweils einer entsprechenden vordefinierbaren ersten und zweiten Länge aufweist, wobei die erste Länge größer als die zweite Länge ist,
eine Lichtquelle, die dazu ausgebildet ist, Licht mit einer im Betrieb einstellbaren Gesamtintensität auszustrahlen, Licht mit einem im Rahmen einer Kalibrierung einstellbaren, ersten Intensitätsanteil in die erste optische Messstrecke einzustrahlen und Licht mit einem im Rahmen einer Kalibrierung einstellbaren, zweiten Intensitätsanteil in die zweite optische Messstrecke einzustrahlen, und
mindestens einen ersten und einen zweiten Lichtdetektor,
wobei der erste Lichtdetektor dazu ausgebildet ist, eine erste Intensität von auf dem ersten Lichtdetektor eintreffendem Licht, das von der Lichtquelle ausgesendet worden ist und die erste optische Messstrecke durchlaufen hat, zu messen und ein erstes elektrisches Ausgangssignal zu erzeugen, das monoton bezüglich der eintreffenden ersten Lichtintensität ist, und der bezüglich der Detektion von Lichtintensität einen ersten Dynamikbereich mit einem darin enthaltenen ersten Intensitätsmessbereich mit optimaler Auflösung zwischen dem Ausgangssignal und der eintreffenden ersten Lichtintensität aufweist,
wobei die Messvorrichtung dazu ausgelegt ist, dass im Betrieb im Rahmen einer Kalibration für eine zu erwartende erste Konzentration der Substanz in dem Fluid und unter Berücksichtigung der voreingestellten Länge der ersten optischen Messstrecke die von der Lichtquelle ausgesendete Gesamtintensität so einstellbar ist, dass die an dem ersten Lichtdetektor eintreffende erste Lichtintensität in dessen ersten Intensitätsmessbereich fällt,
wobei der zweite Lichtdetektor dazu ausgebildet ist, eine zweite Intensität von auf dem zweiten Lichtdetektor eintreffendem Licht, das von der Lichtquelle ausgesendet worden ist und die zweite optische Messstrecke durchlaufen hat, zu messen und ein zweites elektrisches Ausgangssignal zu erzeugen, das monoton bezüglich der eintreffenden zweiten Lichtintensität ist.

Gemäß dem ersten Aspekt der Erfindung kann so die Messvorrichtung dazu ausgelegt sein, dass im Betrieb unter Berücksichtigung der voreingestellten Länge der zweiten optischen Messstrecke und der im Rahmen der Kalibrierung der Lichtquelle eingestellten Gesamtintensität die zweite Messstrecke zusammen mit dem zweiten Lichtdetektor zum Messen einer zweiten Konzentration der Substanz in dem Fluid besser ausgelegt sein als der erste Lichtdetektor, wobei die zweite Konzentration größer als die erste Konzentration ist.

Gemäß einem zweiten Aspekt der Erfindung wird eine zweite fotometrische Messvorrichtung bereitgestellt zum Messen einer optischen Absorbanz eines in einem Fluidbehälter enthaltenen oder in einer Fluidleitung strömenden Fluids, wie etwa eines in einem Dialysatabfluss einer Dialysemaschine strömenden Dialysats, wobei das Fluid entlang der optischen Messstrecke aufgrund einer zeitlich variablen Zusammensetzung oder einer zeitlich variierbaren bzw. variablen Konzentration von einer oder mehreren in dem Fluid enthaltenen, z.B. gelösten oder mitgeführten, Substanzen eine zeitlich variierende Absorbanz aufweisen kann. Die Messvorrichtung gemäß dem zweiten Aspekt der Erfindung kann eines, mehrere oder alle der folgenden Merkmale umfassen:
den Fluidbehälter oder die Fluidleitung, der oder die mindestens eine durch das Fluid verlaufende optische Messstrecke mit einstellbarer Länge aufweist,
eine Lichtquelle, die dazu ausgebildet ist, Licht mit einer im Betrieb einstellbaren Intensität in die optische Messstrecke einzustrahlen, und
mindestens einen Lichtdetektor, der dazu ausgebildet ist, eine Intensität von auf dem Lichtdetektor eintreffendem Licht, das von der Lichtquelle ausgesendet worden ist und die optische Messstrecke durchlaufen hat, zu messen und ein elektrisches Ausgangssignal zu erzeugen, das monoton bezüglich der eintreffenden Lichtintensität ist, und der bezüglich der Detektion von Lichtintensität einen Dynamikbereich mit einem darin enthaltenen Intensitätsmessbereich mit optimaler Auflösung und insbesondere Linearität zwischen dem Ausgangssignal und der eintreffenden Lichtintensität aufweist,
wobei die Messvorrichtung dazu ausgebildet ist, dass im Betrieb für eine vorbestimmte, von der Lichtquelle ausgesendete Lichtintensität und eine zu erwartende Konzentration der Substanz in dem Fluid die Länge der mindestens einen optischen Messstrecke durch das Fluid so einstellbar ist, dass die an dem Lichtdetektor eintreffende Lichtintensität in dessen Intensitätsmessbereich fällt.

Gemäß einem dritten Aspekt der Erfindung wird ein Verfahren bereitgestellt zum Messen einer optischen Absorbanz eines in einem Fluidbehälter enthaltenen oder in einer Fluidleitung strömenden Fluids, wie etwa eines in einem Dialysatabfluss einer Dialysemaschine strömenden Dialysats, wobei das Fluid entlang der optischen Messstrecke aufgrund einer zeitlich variablen Zusammensetzung oder einer zeitlich variierbaren bzw. variablen Konzentration von einer oder mehreren in dem Fluid enthaltenen, z.B. gelösten oder mitgeführten, Substanzen eine zeitlich variierende Absorbanz aufweisen kann. Dieses Verfahren kann einen, mehrere oder alle der folgenden Schritte umfassen:
a) Bereitstellen eines Fluidbehälters oder einer Fluidleitung, der eine erste durch das Fluid verlaufende optische Messstrecke mit einer vordefinierten ersten Länge und eine zweite durch das Fluid verlaufende optische Messstrecke mit einer vordefinierten zweiten Länge aufweist, wobei die erste Länge größer als die zweite Länge ist,
b) Einstrahlen von Licht mit einem ersten Intensitätsanteil in die erste optische Messstrecke und von Licht mit einem zweiten Intensitätsanteil in die zweite optische Messstrecke mittels einer Lichtquelle,
c) Messen einer ersten Intensität von Licht, das von der Lichtquelle ausgesendet worden ist und die erste optische Messstrecke durchlaufen hat, mittels eines ersten Lichtdetektors, der dazu ausgebildet ist, ein erstes elektrisches Ausgangssignal zu erzeugen, das monoton bezüglich der eintreffenden ersten Lichtintensität ist, und der bezüglich der Detektion von Lichtintensität einen ersten Dynamikbereich mit einem darin enthaltenen ersten Intensitätsmessbereich mit optimaler Auflösung und insbesondere Linearität zwischen dem Ausgangssignal und der eintreffenden ersten Lichtintensität aufweist,
d) im Rahmen einer Kalibration, Einstellen der von der Lichtquelle abgestrahlten Gesamtlichtintensität, so dass für eine erste, insbesondere relativ niedrige Konzentration der Substanz in dem Fluid und unter Berücksichtigung der voreingestellten Länge der ersten optischen Messstrecke die an dem ersten Lichtdetektor eintreffende erste Lichtintensität in dessen ersten Messbereich fällt, und
e) mittels des ersten Lichtdetektors, Messen der ersten Intensität bzw. Erfassen eines zeitlichen Verlaufs der ersten Intensität von Licht, das von der Lichtquelle ausgesendet worden ist und die erste optische Messstrecke durchlaufen hat, als Maß für die optische Absorbanz des entlang der ersten optischen Messstrecke vorhandenen Fluids, und gleichzeitig dazu, mittels eines zweiten Lichtdetektors, Messen der zweiten Intensität bzw. Erfassen eines zeitlichen Verlaufs einer zweiten Intensität von Licht, das von der Lichtquelle ausgesendet worden ist und die zweite optische Messstrecke durchlaufen hat, als Maß für die optische Absorbanz des entlang der zweiten optischen Messstrecke vorhandenen gleichartigen Fluids.

Selbstverständlich kann dieses Verfahren ferner den folgenden Schritt umfassen:
wenn im Betrieb eine erste, z.B. relativ niedrige Konzentration der Substanz in dem Fluid vorliegt, Messen der optischen Absorbanz auf der Grundlage der mit dem ersten Lichtdetektor gemessenen Intensität von Licht, das die erste optische Messstrecke durchlaufen hat, und
wenn im Betrieb eine zweite, z.B. relativ hohe Konzentration der Substanz in dem Fluid vorliegt, wobei die zweite Konzentration größer als die erste Konzentration ist, Messen der optischen Absorbanz auf der Grundlage der mit dem zweiten Lichtdetektor gemessenen Intensität von Licht, das die zweite optische Messstrecke durchlaufen hat.

Gemäß einem vierten Aspekt der Erfindung wird ein Verfahren bereitgestellt zum Messen einer optischen Absorbanz eines z.B. in einem Fluidbehälter enthaltenen oder in einer Fluidleitung strömenden Fluids, wie etwa eines in einem Dialysatabfluss einer Dialysemaschine strömenden Dialysats, wobei die optische Absorbanz definiert ist als das mathematische Produkt einer für eine in dem Fluid enthaltenen Substanz spezifischen, wellenlängenabhängigen molaren Absorptionskoeffizienten, einer substanzspezifischen Konzentration und einer Länge einer durch das Fluid verlaufenden optischen Messstrecke, und wobei das Fluid entlang der optischen Messstrecke aufgrund einer zeitlich variablen Zusammensetzung oder einer zeitlich variierbaren bzw. variablen Konzentration von einer oder mehreren in dem Fluid enthaltenen, z.B. gelösten oder mitgeführten, Substanzen eine zeitlich variierende Absorbanz aufweisen kann, welches Verfahren eines, mehrere oder alle der folgenden Schritte umfassen kann:
a) Bereitstellen eines Fluidbehälters oder einer Fluidleitung, bei dem oder der mindestens die Länge einer durch das Fluid verlaufende optische Messstrecke eingestellt werden kann,
b) Einstrahlen von Licht mit einem ersten Intensitätsanteil in die optische Messstrecke mit einer eingestellten ersten Länge mittels einer Lichtquelle,
c) Messen einer ersten Intensität von Licht, das von der Lichtquelle ausgesendet worden ist und die optische Messstrecke mit der ersten Länge durchlaufen hat mittels eines Lichtdetektors, der dazu ausgebildet ist, ein elektrisches Ausgangssignal zu erzeugen, das monoton bezüglich der eintreffenden Lichtintensität ist, und der bezüglich der Detektion von Lichtintensität einen Dynamikbereich mit einem darin enthaltenen Intensitätsmessbereich mit optimaler Auflösung und insbesondere Linearität zwischen dem Ausgangssignal und der eintreffenden Lichtintensität aufweist,
d) im Rahmen einer Kalibration, Einstellen der von der Lichtquelle abgestrahlten Gesamtlichtintensität, so dass für eine erste Konzentration der Substanz in dem Fluid und unter Berücksichtigung der eingestellten ersten Länge der optischen Messstrecke die an dem Lichtdetektor eintreffende Lichtintensität in dessen Intensitätsmessbereich fällt,
e) Messen der Intensität bzw. Erfassen eines zeitlichen Verlaufs der Intensität von auf dem Lichtdetektor eintreffendem Licht als Maß für die Absorbanz des in der optischen Messstrecke mit der eingestellten ersten Länge vorhandenen Fluids,
f) wenn die an dem Lichtdetektor eintreffende Lichtintensität nicht mehr in dessen Messbereich fällt, neu Einstellen der Länge der optischen Messstrecke auf eine zweite Länge (I₂), so dass die an dem Lichtdetektor eintreffende Lichtintensität wieder in dessen Messbereich fällt, und
g) Messen der Intensität bzw. Erfassen eines zeitlichen Verlaufs der Intensität von auf dem Lichtdetektor eintreffendem Licht als Maß für die Absorbanz des entlang der optischen Messstrecke vorhandenen Fluids mit der neu-eingestellten Länge.

Selbstverständlich kann dieses Verfahren ferner den folgenden Schritt umfassen:
h) Wiederholen der Schritte f) und g) während einer einstellbaren Messdauer zum Erfassen eines zeitlichen Verlaufs der optischen Absorbanz des entlang der optischen Messstrecke vorhandenen Fluids mit Berücksichtigung der jeweiligen eingestellten Länge der optischen Messstrecke.

In den Vorrichtungen nach dem ersten und zweiten Aspekt sowie in dem Verfahren nach dem dritten und vierten Aspekt der Erfindung ist die optische Absorbanz definiert als das mathematische Produkt einer für eine in dem Fluid enthaltenen Substanz spezifischen, wellenlängenabhängigen molaren Absorptionskoeffizienten, einer substanzspezifischen Konzentration und der Länge der durch das Fluid verlaufenden optischen Messstrecke.

In der Messvorrichtung gemäß dem ersten Aspekt der Erfindung kann bei dem ersten Lichtdetektor das erste elektrische Ausgangssignal streng monoton bezüglich der eintreffenden ersten Lichtintensität sein. Ferner kann sich der erste Intensitätsmessbereich neben der optimalen Auflösung auch durch eine Linearität zwischen dem Ausgangssignal und der eintreffenden ersten Lichtintensität auszeichnen.

In der Messvorrichtung gemäß dem ersten Aspekt der Erfindung kann bei dem zweiten Lichtdetektor das zweite elektrische Ausgangssignal streng monoton bezüglich der eintreffenden zweiten Lichtintensität sein. Ferner kann der zweite Lichtdetektor bezüglich der Detektion von Lichtintensität einen zweiten Dynamikbereich mit einem darin enthaltenen zweiten Intensitätsmessbereich mit optimaler Auflösung und insbesondere Linearität zwischen dem Ausgangssignal und der eintreffenden zweiten Lichtintensität aufweisen.

Die Messvorrichtung gemäß dem ersten Aspekt der Erfindung kann dazu ausgelegt sein, dass im Betrieb der erste Lichtdetektor in einem ersten Intensitätsmessintervall und gleichzeitig der der zweite Lichtdetektor in einem zweiten Intensitätsmessintervall betrieben werden kann.

Die Messvorrichtung gemäß dem ersten Aspekt der Erfindung kann so ausgelegt sein, dass im Betrieb die erste optische Messstrecke zusammen mit dem ersten Lichtdetektor und dessen ersten Intensitätsmessbereich zum Messen einer ersten Konzentration der Substanz in dem Fluid ausgelegt ist, und dass die zweite optische Messstrecke zusammen mit dem zweiten Lichtdetektor und dessen zweiten Intensitätsmessbereich zum Messen einer zweiten Konzentration der Substanz in dem Fluid ausgelegt ist, wobei die zweite Konzentration größer als die erste Konzentration ist. Die erste Konzentration kann eine relativ niedrige Konzentration und die zweite Konzentration kann eine relativ hohe Konzentration der Substanz in dem Fluid sein.

In der Messvorrichtung Messvorrichtung gemäß dem ersten Aspekt der Erfindung kann die Lichtquelle eine Lichtstrahlungsaufteilungseinrichtung umfassen, die dazu ausgebildet ist, einen im Rahmen einer Kalibrierung einstellbaren ersten Teil der Gesamtintensität in die erste optische Messstrecke einzustrahlen und einen im Rahmen einer Kalibrierung einstellbaren zweiten Teil der Gesamtintensität in die zweite optische Messstrecke einzustrahlen.

Dabei kann die Lichtstrahlungsaufteilungseinrichtung einen teildurchlässigen, z.B. einen halbdurchlässigen Spiegel umfassen, der so angeordnet ist, dass er den ersten oder den zweiten Teil reflektiert und den zweiten oder den ersten Teil durchlässt.

In einer ersten Ausführungsform der Messvorrichtung gemäß dem ersten Aspekt der Erfindung kann die Lichtquelle folgendes umfassen:
einen ersten Lichtleiter, wie etwa eine Lichtleitfaser, mit einem ersten Ende und einem zweiten Ende, wobei der von dem teildurchlässigen Spiegel reflektierte erste oder zweite Teil an dem ersten Ende in den ersten Lichtleiter einkoppelbar ist, und wobei das zweite Ende an einen Anfang der ersten optischen Messstrecke optisch ankoppelbar ist, und
einen zweiten Lichtleiter, wie etwa eine Lichtleitfaser, mit einem ersten Ende und einem zweiten Ende, wobei durch das erste Ende der von dem teildurchlässigen Spiegel durchgelassene zweite oder erste Teil in den zweiten Lichtleiter einkoppelbar ist und wobei das zweite Ende an einen Anfang der zweiten optischen Messstrecke optisch ankoppelbar wird.

In einer dazu alternativen zweiten Ausführungsform der Messvorrichtung gemäß dem ersten Aspekt der Erfindung kann die Lichtstrahlungsaufteilungseinrichtung folgendes umfassen:
einen ersten Lichtleiter, wie etwa eine Lichtleitfaser, mit einem ersten und einem zweiten Ende, wobei das zweite Ende an einen Anfang der ersten optischen Messstrecke optisch ankoppelbar ist,
einen zweiten Lichtleiter, wie etwa eine Lichtleitfaser, mit einem ersten und einem zweiten Ende, wobei das zweite Ende an einen Anfang der zweiten optischen Messstrecke optisch ankoppelbar ist, und
eine Lichtaufteilungseinrichtung mit einem Lichteingang sowie einem ersten und einem zweiten Lichtausgang, die dazu ausgebildet ist, in den Lichteingang eingestrahltes Licht aufzuteilen in einen durch den ersten Lichtausgang austretenden ersten Teil und einen durch den zweiten Lichtausgang austretenden zweiten Teil, wobei zumindest ein Teil der von der Lichtquelle ausgestrahlten Gesamtintensität durch den Lichteingang in die Lichtaufteilungseinrichtung einkoppelbar ist, der erste Lichtausgang an das erste Ende des ersten Lichtleiters optisch ankoppelbar ist und der zweite Lichtausgang an das erste Ende des zweiten Lichtleiters ankoppelbar ist.

In dieser zweiten Ausführungsform kann der erste Lichtausgang der Lichtaufteilungseinrichtung mit einer Lichteintrittsfacette des ersten Lichtleiters und der zweite Lichtausgang der Lichtaufteilungseinrichtung mit einer Lichteintrittsfacette des zweiten Lichtleiters optisch gekoppelt sein.

In der Messvorrichtung gemäß dem ersten Aspekt der Erfindung kann die erste optische Messstrecke eine im Rahmen einer Kalibrierung einstellbare erste Länge und die zweite optische Messstrecke eine ebenfalls im Rahmen einer Kalibrierung einstellbare, von der ersten Länge unterschiedliche zweite Länge aufweisen.

Der erste Lichtdetektor kann an ein Ende der ersten optischen Messstrecke optisch ankoppelbar sein und insbesondere eine erste Licht detektierende Einheit, wie etwa eine Photodiode, umfassen. Auch kann der zweite Lichtdetektor an ein Ende der zweiten optischen Messstrecke optisch ankoppelbar sein und insbesondere eine zweite Licht detektierende Einheit, wie etwa eine Photodiode, umfassen.

Alternativ dazu kann der erste Lichtdetektor eine erste Licht detektierende Einheit, wie etwa eine Photodiode und einen ersten Lichtleiter, wie etwa eine Lichtleitfaser, mit einem ersten und einem zweiten Ende umfassen, wobei das erste Ende an ein Ende der ersten optischen Messstrecke und das zweite Ende des ersten Lichtleiters an die erste Licht detektierende Einheit optisch ankoppelbar ist. Überdies kann der zweite Lichtdetektor eine zweite Licht detektierende Einheit, wie etwa eine Photodiode und einen zweiten Lichtleiter, wie etwa eine Lichtleitfaser, mit einem ersten und einem zweiten Ende umfassen, wobei das erste Ende an ein Ende der zweiten optischen Messstrecke und das zweite Ende des zweiten Lichtleiters an die zweite Licht detektierende Einheit optisch ankoppelbar ist.

In der Messvorrichtung gemäß dem zweiten Aspekt der Erfindung können der Fluidbehälter oder die Fluidleitung Mittel zum Einstellen der Länge der optischen Messstrecke umfassen.

In einer ersten Ausführungsform können diese Einstellmittel zwei einander gegenüberliegend angeordnete, die optische Messstrecke begrenzende Wände bzw. Wandungsabschnitte des Fluidbehälters oder der Fluidleitung umfassen, von denen mindestens eine oder beide in einer Richtung parallel zur Richtung der optischen Messstrecke verschiebbar ausgebildet sind, insbesondere in Richtung zu der/dem jeweils anderen Wand bzw. Wandungsabschnitt oder in Richtung weg davon.

In der ersten Ausführungsform kann die/der mindestens eine verschiebbare Wand bzw. Wandungsabschnitt des Fluidbehälters oder der Fluidleitung ein Teil eines verschiebbaren Kolbens sein. Dabei kann die Lichtquelle an bzw. in der mindestens einen Wand an einen Anfang der optischen Messstrecke optisch ankoppelbar sein. Dabei kann ferner der Lichtdetektor an bzw. in einer der Wand mit dem Lichtdetektor gegenüberliegenden, nicht verschiebbaren Wand an ein Ende der optischen Messstrecke optisch ankoppelbar sein. Alternativ dazu kann der Lichtdetektor kann an bzw. in der mindestens einen verschiebbaren Wand an ein Ende der optischen Messstrecke optisch ankoppelbar sein. Dabei kann ferner die Lichtquelle an bzw. in einer der verschiebbaren Wand gegenüberliegenden, nicht verschiebbaren Wand an einen Anfang der optischen Messstrecke optisch ankoppelbar sein.

In einer zweiten Ausführungsform können diese Einstellmittel zwei einander gegenüberliegend und in einer Richtung parallel zur Richtung der optischen Messstrecke verschiebbar angeordnete Begrenzungsbacken und einen zwischen den Begrenzungsbacken angeordneten Umfangswandungsabschnitt des Fluidbehälters mit einer elastisch verformbaren in einem entspannten Zustand im Wesentlichen zylinderförmigen Wand umfassen. Dabei sind die Begrenzungsbacken in einander gegenüberliegend angeordneten, die optische Messstrecke begrenzenden Wandungsbereichen mit einer Außenseite des Umfangswandungsabschnitts in Kontakt, so dass die Länge der optischen Messstrecke durch Verändern des Abstands der Spannbacken einstellbar ist.

In der Messvorrichtung gemäß dem zweiten Aspekt der Erfindung kann die Lichtquelle an einen Anfang der optischen Messstrecke optisch ankoppelbar sein. Dabei kann sie eine Licht emittierende Einheit, wie etwa eine LED, umfassen.

Alternativ dazu kann die Lichtquelle eine Licht emittierende Einheit, wie etwa eine LED, und einen Lichtleiter, wie etwa eine Lichtleitfaser, mit einem ersten und einem zweiten Ende umfassen. Dabei kann das erste Ende des Lichtleiters an die Licht emittierende Einheit und das zweite Ende des Lichtleiters an einen Anfang der optischen Messstrecke optisch ankoppelbar sein.

In der Messvorrichtung gemäß dem zweiten Aspekt der Erfindung kann der Lichtdetektor an ein Ende der optischen Messstrecke optisch ankoppelbar sein. Dabei kann er eine Licht detektierende Einheit, wie etwa eine Photodiode, umfassen.

Alternativ dazu kann der Lichtdetektor eine Licht detektierende Einheit, wie etwa eine Photodiode, und einen Lichtleiter, wie etwa eine Lichtleitfaser, mit einem ersten und einem zweiten Ende umfassen. Dabei kann das erste Ende des Lichtleiters an ein Ende der optischen Messstrecke und das zweite Ende des Lichtleiters an die Licht detektierende Einheit optisch ankoppelbar sein.

Die Messvorrichtung gemäß dem ersten und dem zweiten Aspekt der Erfindung kann einen Referenz-Lichtdetektor umfassen, der optisch an die Lichtquelle gekoppelt ist und der dazu ausgebildet ist, ein zu der von Lichtquelle abgestrahlten Gesamtlichtintensität proportionales, elektrisches Ausgangssignal zu erzeugen. Dieses Ausgangssignal kann als Referenzwert für die von Lichtquelle abgestrahlte Gesamtlichtintensität verwendet werden. In dieser Ausgestaltung kann die Messvorrichtung ferner Mittel zum Regeln der von der Lichtquelle abgestrahlten Gesamtlichtintensität auf ein einstellbares Niveau umfassen, welche Mittel das Ausgangssignals des Referenz-Lichtdetektors, insbesondere als ein Eingangssignal, benutzen.

Die Messvorrichtung gemäß dem ersten Aspekt der Erfindung kann mindestens ein mechanisches Mittel zum Einstellen einer vorbestimmten Solllänge der ersten optischen Messstrecke und/oder der zweiten optischen Messstrecke umfassen.

Entsprechend kann die Messvorrichtung gemäß dem zweiten Aspekt der Erfindung mindestens ein mechanisches Mittel zum Einstellen einer vorbestimmten Solllänge der optischen Messstrecke mit einstellbarer Länge umfassen.

Die Messvorrichtungen gemäß dem ersten und dem zweiten Aspekt der Erfindung können eine spektralfotometrische Messvorrichtung sein, die dazu ausgebildet ist, die optische Absorbanz in einem vorbestimmten Wellenlängenbereich oder in einem einstellbaren oder einem durchstimmbaren Wellenlängenbereich zu messen.

Die Messvorrichtungen gemäß dem ersten und dem zweiten Aspekt der Erfindung können in einer Blutbehandlungsvorrichtung, einschließlich etwa einer Dialysemaschine eingesetzt werden bzw. enthalten sein.

Eine Dialysemaschine kann folgendes umfassen:
einen an einen Patienten anschließbaren extrakorporalen Blutkreislauf, in dem im Betrieb Blut des Patienten strömt, einen Dialysatkreislauf mit einer ein aufbereitetes Dialysat führenden Dialysatzufuhrleitung und einer Dialysatabfuhrleitung, und eine Dialysatoreinrichtung mit einer Membranfiltereinrichtung, wobei die Dialysatzufuhrleitung und die Dialysatabfuhrleitung über eine Membran der Membranfiltereinrichtung mit dem extrakorporalen Blutkreislauf so koppelbar ist, dass ein Substanzaustausch durch die Membran stattfinden kann, wobei die Membran dazu ausgebildet ist, im Betrieb in dem im Blutkreislauf geführten Blut mitgeführte bzw. darin gelöste Substanzen, wie etwa harnpflichtige Substanzen und/oder urämische Toxine, durchzulassen und dem aufbereiteten Dialysat hinzu zu mischen, wobei in der Dialysatabfuhrleitung eine Mischung aus dem aufbereiteten Dialysat und den durch die Membran durchgelassenen Substanzen aus dem Blut geführt wird.

In einer derartigen Dialysemaschine kann die Messeinrichtung gemäß dem ersten bzw. zweiten Aspekt der Erfindung an bzw. in der Dialysatabfuhrleitung angeordnet sein. Dabei kann die Messeinrichtung dazu ausgebildet sein, eine optische Absorbanz einer in der Dialysatabfuhrleitung mitgeführten bzw. gelösten Substanz aus dem Blut des Patienten zu messen.

### Kurze Beschreibung der Figuren

Die Erfindung wird im Folgenden anhand von in den beigefügten Figuren gezeigten Ausführungsformen der fotometrischen Messvorrichtung gemäß dem ersten bzw. zweiten Aspekt der Erfindung näher beschreiben. Es zeigen:
Figur 1 ein Flussschema eines Blut- und Dialysatkreislaufs in einem Dialysebetrieb einer Blutbehandlungsvorrichtung mit einer erfindungsgemäßen fotometrischen Messvorrichtung, wobei ein Patient an den extrakorporalen Blutkreislauf angeschlossen ist;
Figur 2 eine schematische Ansicht der in Fig. 1 gezeigten Blutbehandlungsvorrichtung;
Figur 3 Emissionsspektren einer Lichtquelle einer erfindungsgemäßen fotometrischen Messvorrichtung, die bei unterschiedlichen Lichtintensitäten gemessen worden sind;
Figur 4 schematische Darstellungen einer Ausführungsform einer fotometrischen Messvorrichtung gemäß dem zweiten Aspekt der Erfindung mit unterschiedlich lang eingestellten optischen Messstrecken;
Figur 5 eine schematische Darstellung einer ersten Ausführungsform einer fotometrischen Messvorrichtung gemäß dem ersten Aspekt der Erfindung;
Figur 6 eine schematische Darstellung einer zweiten Ausführungsform einer fotometrischen Messvorrichtung gemäß dem ersten Aspekt der Erfindung;
Figur 7 eine schematische Darstellung einer dritten Ausführungsform einer fotometrischen Messvorrichtung gemäß dem ersten Aspekt der Erfindung, wobei ein jeweilige Lichtquelle direkt an eine jeweilige Fluidleitung mit einer ersten bzw. zweiten optischen Messstrecke operativ gekoppelt ist;
Figur 8 eine schematische Darstellung einer vierten Ausführungsform einer fotometrischen Messvorrichtung gemäß dem ersten Aspekt der Erfindung, wobei ein jeweilige Lichtquelle direkt an einen jeweiligen Fluidbehälter mit einer ersten bzw. zweiten optischen Messstrecke operativ gekoppelt ist; und
Figur 9 eine schematische Darstellung einer fünften Ausführungsform einer fotometrischen Messvorrichtung gemäß dem ersten Aspekt der Erfindung, wobei ein jeweilige Lichtquelle direkt an eine Fluidleitung bzw. einen Fluidbehälter mit einer ersten bzw. zweiten optischen Messstrecke operativ gekoppelt ist.

### Ausführungsformen der Erfindung

Wie eingangs bereits erwähnt, kann eine photometrische Messvorrichtung 200, 300 gemäß dem ersten bzw. zweiten Aspekt der Erfindung in einer Blutbehandlungsvorrichtung, wie z. B. der Dialysemaschine 100 zum Durchführen einer Hämodialysebehandlung eines Patienten 10 verwendet werden. Die Dialysemaschine 100 umfasst ein Maschinengehäuse 102, einen in Bezug auf den Patienten 10 extrakorporal geführten Blutkreislauf 20, der, wie in Fig. 2 angedeutet, im Wesentlichen außerhalb des Maschinengehäuses 102 angeordnet ist, einen Dialysatkreislauf 60, der im Wesentlichen innerhalb des Maschinengehäuses 102 angeordnet ist, und eine Dialysatoreinrichtung 50 mit einer darin angeordneten Membranfiltereinrichtung 56 mit einer Membran, durch die ein Stoffaustausch zwischen dem Blut und dem Dialysat stattfindet. Die Leitungen des extrakorporalen Blutkreislaufs 20 und die Dialysatoreinrichtung 50 sind als Einmalartikel ausgebildet und im Wesentlichen aus Kunststoff hergestellt.

Der extrakorporale Blutkreislauf 20 umfasst einen arteriellen Zugang 22 zum Arteriensystem des Patienten 10, ein arterielles Schlauchsystem 24, das am arteriellen Zugang 22 beginnt und arterielles Blut zu einem Bluteinlass 34 in den Blutbereich 52 der Dialysatoreinrichtung 50 leitet, den Blutbereich 52 selbst, und ein venöses Schlauchsystem 44, das durch einen Blutauslass 36 des Blutbereichs 52 austretendes, gereinigtes Blut zu einem venösen Zugang 48 am Patienten zurück in den Körper des Patienten 10 leitet. In dem arteriellen Schlauchsystem 24 sind ein arterieller Luftfänger 26, ein erster Drucksensor 28 zum Messen eines arterienseitigen Drucks, eine arterielle Blutpumpe 30, die vorzugsweise und wie in Fig. 2 als eine z. B. an einer Frontseite des Maschinengehäuses 102 angeordnete Schlauchpumpe ausgebildet ist, und ein zweiter Drucksensor 32 zum Messen eines am Bluteinlass 34 anliegenden Blutbereichseingangsdrucks seriell hintereinander angeordnet. In dem venösen Schlauchsystem 44 sind ein Ausgleichsbehälter 38 über eine Abzweigung (nicht bezeichnet), ein dritter Drucksensor 42 zum Messen eines im Wesentlichen am venösen Zugang 48 gebildeten venösen Rückstaudrucks und ein venöser Luftfänger 46 seriell hintereinander angeordnet.

Der Dialysatkreislauf 60 beginnt bei einer Dialysataufbereitung 62, indem patientenspezifisch einem stromsterilisierten Wasser in jeweils dem patientenspezifisch dosierbarer Menge Dialysataufbereitungsmittel wie etwa Bicarbonat und ein Säurekonzentrat zugemischt werden, eine Flusspumpe 64 im Dialysatzufluss zum Einstellen eines patienten- bzw. behandlungsspezifischen Dialysatstroms, ein steuerbares erstes Bypassventil 66, das das aufbereitete Dialysat wahlweise durch eine Dialysatzufuhrleitung 72 einem Dialysateinlass 74, im weiteren Verlauf dem Dialysatbereich 54 der Dialysatoreinrichtung 50, und nach dem Stoffaustausch durch einen Dialysatauslass 76 aus dem Dialysatbereich 54 zu einer Dialysatabfuhrleitung 78 zugeführt werden kann oder alternativ durch eine Bypassleitung 68 am Dialysatbereich 54 vorbeigeleitet werden kann. Auf der Dialysatabfuhrseite umfasst der Dialysatkreislauf 60 ein zweites Bypassventil 70, in dem die Bypassleitung 68 und der durch den Dialysatbereich 54 führende Leitungsabschnitt zusammengeführt sind und wahlweise einer dieser beiden Leitungen den weiteren Verlauf der Dialysatabfuhr zugeführt wird, sowie die im weiteren Verlauf der Dialysatabfuhr eingeschlauchte photometrische Messvorrichtung 200, 300 gemäß dem ersten bzw. zweiten Aspekt der Erfindung und schließlich ein Dialysatabfluss 82, der als Sammelcontainer und insbesondere als Einmalartikel ausgebildet sein kann.

Wie in Fig. 2 angedeutet, umfasst die Dialysemaschine 100 ferner eine Ein-/Ausgabeeinrichtung 110, die z. B. als ein Touchscreen ausgebildet sein kann und an einer für einen Bediener sichtbaren Vorderseite des Maschinengehäuses 102 angeordnet ist, und eine Steuer- und Regelungseinheit 120, die zum Steuern und Regeln von Funktionen der Dialysemaschine zum Betreiben des Dialysatkreislaufs 60 und zum Steuern von Funktionen im extrakorporalen Blutkreislauf 20 ausgebildet ist. Die Steuer- und Regelungseinheit 120 umfasst auch Einheiten, die zum Ansteuern der Ein-/Ausgabeeinrichtung 110, insbesondere zum Steuern und Einholen von Bedienereingaben und zum Steuern von Anzeigebildern bzw. Anzeigebildschirmen der Ein-/Ausgabeeinrichtung 110 ausgebildet sind. Im Zusammenspiel mit der Steuer- und Regelungseinheit 120 kann ein Ausgabeteil der Ein-/Ausgabeeinrichtung 110 unter anderem Funktionsparameter des Betriebs des Dialysatkreislaufs 60, Funktionsparameter des Blutkreislaufs 20 und Messergebnisse der Messeinrichtung 200 bzw. 300 einschließlich etwa dem zeitlichen Verlauf bzw. den zeitlichen Verläufen der gemessenen optischen Absorbanz bzw. in skalierter Form der ermittelten Konzentrationen der im Fluid (Dialysat) enthaltenen zu messenden Substanzen.

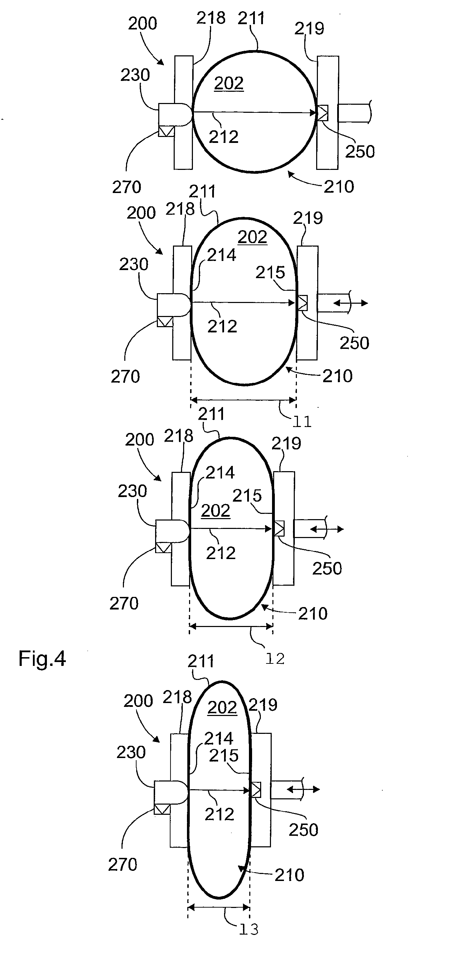

In Fig. 4 ist eine photometrische Messvorrichtung 200 gemäß dem zweiten Aspekt der Erfindung in vier verschiedenen Zuständen mit unterschiedlich lang eingestellten optischen Messstrecken 212 schematisch dargestellt. Die Messvorrichtung 200 umfasst einen Fluidbehälter 210 mit dem darin enthaltenen Fluid 202 mit den darin enthaltenen bzw. gelösten, nachzuweisenden Substanzen, eine Lichtquelle 230, die dazu ausgebildet ist, sich mit einer im Betrieb einstellbaren Intensität in die optische Messstrecke 212 einzustrahlen, mindestens einen Lichtdetektor 250, der dazu ausgebildet ist, eine Intensität von auf dem Lichtdetektor 250 eintreffenden Licht, das von der Lichtquelle 230 ausgesendet worden ist und die optische Messstrecke 212 durchlaufen hat, zu messen und ein elektrisches Ausgangssignal zu erzeugen, das streng monoton bezüglich der eintreffenden Lichtintensität ist, und der bezüglich der Detektion von Lichtintensität einen Dynamikbereich mit einem darin enthaltenen Intensitätsmessbereich mit optimaler Auflösung und Linearität zwischen dem Ausgangssignal und der eintreffenden Lichtintensität aufweist, einen Referenz-Lichtdetektor 270, der optisch an die Lichtquelle 230 gekoppelt ist und der dazu ausgebildet ist, ein zu der von der Lichtquelle 230 abgestrahlte Gesamtlichtintensität proportionales, elektrisches Ausgangssignal zu erzeugen, und Mittel 213`, 213" zum Einstellen der Länge L₁, L₂, L₃ der optischen Messstrecke 212.

Die Messvorrichtung 200 gemäß dem zweiten Aspekt der Erfindung ist dadurch gekennzeichnet, dass die Länge L₁, L₂, L₃ der optischen Messstrecke 212 durch das Fluid 202 steuerbar einstellbar ist. Dazu umfasst die Messvorrichtung 200 Mittel 214, 215, 218, 219 zum Einstellen der Länge der optischen Messstrecke 212. In der in Fig. 4 gezeigten Ausführungsform umfassen die Einstellmittel zwei einander gegenüberliegend und in eine Richtung parallel zur Richtung der optischen Messstrecke 212 verschiebbar angeordnete Begrenzungsbacken 218, 219 und einen zwischen den Begrenzungsbacken 218, 219 angeordneten Umfangswandungsabschnitt 211 des Fluidbehälters 210 mit einer elastisch verformbaren, in ihrem entspannten Zustand zum Beispiel im Wesentlichen zylinderförmigen Wand, wie im ersten Zustand bzw. dem obersten der vier Skizzen in Fig. 4 gezeigt. Die Begrenzungsbacken 218, 219 sind in einander gegenüberliegend angeordneten, die optische Messstrecke 212 begrenzenden Wandungsbereichen mit einer Außenseite des Umfangswandungsabschnitts 211 in Kontakt, so dass die Länge L₁, L₂, L₃ der optischen Messstrecke 212 durch Verändern des Abstands der Spannbacken 218, 219 einstellbar ist, wie in dem zweiten bis vierten Zustand bzw. den unteren drei Teilfiguren der Fig. 4 gezeigt.

Die Lichtquelle 230 umfasst eine Licht emittierende Einheit, insbesondere eine LED. Die Lichtquelle 230 ist an bzw. in der einen (in Fig. 4 linken) Begrenzungsbacke 218 angeordnet und an einen Anfang der einstellbaren optischen Messstrecke 212 optisch angekoppelt, so dass sie Licht in die optische Messstrecke 212 einstrahlen kann. Die Lichtquelle 230 kann alternativ dazu über eine Lichtleitung (nicht gezeigt) in die Begrenzungsbacke 218 bzw. den Anfang der optischen Messstrecke 212 optisch eingekoppelt sein.

Der Lichtdetektor ist an der gegenüberliegenden (in Fig. 4 rechten) zweiten Begrenzungsbacke 219 angebracht. Er ist an ein Ende der optischen Messstrecke 212 optisch angekoppelt. Er kann eine Licht detektierende Einheit, wie etwa eine Photodiode, umfassen. Alternativ dazu kann der Lichtdetektor 250 auch über eine Lichtleitung (nicht gezeigt) versetzt von der zweiten Begrenzungsbacke 219 angeordnet und mit dem Ende der optischen Messstrecke optisch angekoppelt sein.

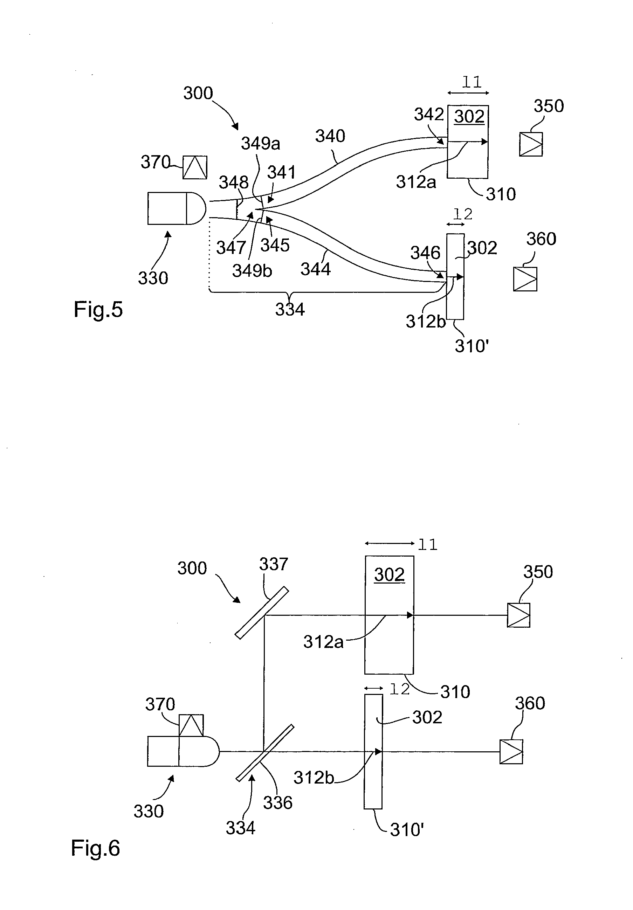

Der Lichtdetektor 250 und ebenso die Lichtdetektoren 350 und 360 in den in den Figuren 5 bis 9 gezeigten Ausführungsformen ist dazu ausgebildet, eine Intensität von dem auf dem Lichtdetektor eintreffenden Licht, das von der Lichtquelle ausgesendet worden ist und die entsprechende optische Messstrecke durchlaufen hat, zu messen und ein erstes elektrisches Ausgangssignal zu erzeugen, das monoton bezüglich der eintreffenden Lichtintensität ist. Dabei weist ein jeweiliger Lichtdetektor eine Kennlinie für das erzeugte elektrische Ausgangssignal als Funktion der eintreffenden Lichtintensität auf, welche Kennlinie vergleichbar der Belichtungskennlinie einer Filmplatte, bei einer relativ niedrigen eintreffenden Lichtintensität zunächst einen nur langsam ansteigenden Verlauf, der steiler wird und einen bis zu einem Wendepunkt zunehmend steiler werdenden und in einen linearen Abschnitt übergehenden Verlauf, der bei weiter zunehmender Lichtintensität wieder weniger steil wird und in eine Sättigung läuft. Zwischen einem aufwärts gekrümmten Abschnitt bei niedrigen Lichtintensitäten und dem bei höheren Lichtintensitäten wieder flacher werdenden Verlauf weist die Detektorkennlinie einen Dynamikbereich mit einem darin enthaltenen ersten Intensitätsmessbereich mit optimaler Auflösung, d. h. z.B. größter Steigung bzw. Zunahme des Ausgangssignals bei zunehmender Lichtintensität mit der größten Steigung, d. h. mit optimaler Auflösung und insbesondere einem linearen Verlauf zwischen dem Ausgangssignal und der eintreffenden Lichtintensität auf. Gemäß einem Grundgedanken der Erfindung soll der Lichtdetektor in dessen Intensitätsmessbereich, d. h. den Bereich der größten Auflösung und besten Linearität, betrieben werden.

Im Betrieb der Messvorrichtung 200 wird zunächst Licht von der Lichtquelle 230 mit einer Anfangsintensität ausgestrahlt und ein erster Intensitätsanteil davon in die optische Messstrecke 212 mit einer voreingestellten ersten Länge, z. B. L₁, eingestrahlt. Der Lichtdetektor 250 misst eine erste Intensität von Licht, das von der Lichtquelle ausgesendet worden ist und die optische Messstrecke 212 mit der ersten Länge durchlaufen hat, wobei entlang der optischen Messstrecke 212 eine erste Konzentration der zu messenden Substanz in dem Fluid 202 vorhanden ist. Die erste Konzentration kann dabei auch sehr gering bzw. null sein. Sodann wird im Rahmen einer Kalibration die von der Lichtquelle 230 abgestrahlte Gesamtlichtintensität so eingestellt, dass für die erste Konzentration der Substanz in dem Fluid 202 und unter Berücksichtigung der eingestellten ersten Länge, z. B. L₁, der optischen Messstrecke 212 die an dem Lichtdetektor 250 eintreffende Lichtintensität in dessen Intensitätsmessbereich fällt. Der Referenz-Lichtdetektor 270 misst ein Signal, das proportional zu dem von der Lichtquelle 230 ausgesendeten Gesamtintensität ist. Nach dem Kalibrieren der ausgesendeten Lichtintensität der Lichtquelle 230 wird das entsprechende Ausgangssignal des Referenz-Lichtdetektors 270 als Referenzsignal verwendet, und beispielsweise als Eingangsgröße einer Regelungseinrichtung zum Regeln der Gesamtausgangslichtleistung der Lichtquelle 230 auf ein konstantes Level zugeführt. Durch das Referenzlichtsignal bzw. diese Regelung auf ein konstantes Ausgangslevel werden Schwankungen der von der Lichtquelle 230 ausgesendeten Lichtintensität, wie sie etwa infolge von Temperatureinflüssen auftreten können, kompensiert. Nach der Kalibration und unter der Lichtintensitätsregelung auf konstantes Level wird dann ein zeitlicher Verlauf der Intensität von auf dem Lichtdetektor 250 eintreffenden Licht als Maß für die Absorbanz des in der optischen Messstrecke mit der eingestellten ersten Länge vorhandenen Fluids gemessen bzw. erfasst. Wenn nun die an dem Lichtdetektor 250 eintreffende Lichtintensität nicht mehr in dessen Messbereich fällt, etwa weil die Konzentration der zu messenden Substanz so stark zugenommen hat, dass der Lichtdetektor 250 außerhalb von dessen Messbereich betrieben wird, wird die Länge der optischen Messstrecke neu eingestellt auf eine zweite Länge, z. B. L₂, die so gewählt ist, dass die an dem Lichtdetektor 250 eintreffende Lichtintensität wieder in dessen Messbereich fällt. Die neu eingestellte zweite Länge wird registriert und fortan wird ein zeitlicher Verlauf der Intensität von auf dem Lichtdetektor 250 eintreffendem Licht als Maß für die Absorbanz des in der optischen Messstrecke 212 mit der neu eingestellten zweiten Länge vorhandene Fluid gemessen bzw. erfasst. Die Schritte des Neueinstellens der Länge der optischen Messstrecke, des Registrierens der neuen Messstrecke und das Messen bzw. Erfassen des zeitlichen Verlaufs der Intensität mit der neu eingestellten Länge der optischen Messstrecke wird während einer einstellbaren Messdauer bei Bedarf wiederholt, wenn die Konzentration der in dem Fluid gelösten Substanz sich so stark verändert, die auf den Lichtdetektor 250 eintreffende Lichtintensität nicht mehr in dessen Messbereich fällt.

Die Figuren 5 und 6 zeigen schematische Darstellungen einer ersten und zweiten Ausführungsform einer photometrischen Messvorrichtung gemäß dem ersten Aspekt der Erfindung. Diesen beiden Ausführungsformen ist gemeinsam, dass die Messvorrichtung 300 Folgendes umfasst: einen Fluidbehälter 310, 310' bzw. eine Fluidleitung, der oder die eine erste und eine zweite durch das Fluid 302 verlaufende optische Messstrecke 312a, 312b mit jeweils einer entsprechenden vordefinierten ersten und zweiten Länge L₁, L₂ aufweist, wobei die erste Länge L₁ größer als die zweite Länge L₂ ist, eine Lichtquelle 330, die dazu ausgebildet ist, Licht mit einer im Betrieb einstellbaren Gesamtintensität auszustrahlen, von dem ausgestrahlten Licht mit einem im Rahmen einer Kalibrierung einstellbaren ersten Intensitätsanteil in die erste optische Messstrecke 312a einzustrahlen und Licht mit einem im Rahmen einer Kalibrierung einstellbaren zweiten Intensitätsanteil in die zweite optische Messstrecke 312b einzustrahlen, einen ersten und einen zweiten Lichtdetektor 350, 360, die dazu ausgebildet sind, eine erste bzw. zweite Intensität von auf dem ersten bzw. zweiten Lichtdetektor 350, 360 eintreffenden Licht, das von der Lichtquelle 330 ausgesendet worden ist und die erste bzw. zweite optische Messstrecke 312a, 312b durchlaufen hat, zu messen und ein entsprechendes erstes bzw. zweites elektrisches Ausgangssignal zu erzeugen, das monoton bezüglich der eintreffenden ersten bzw. zweiten Lichtintensität ist. Dabei umfasst die Lichtquelle 330 ferner eine Lichtstrahlungsaufteilungseinrichtung 334, die dazu ausgebildet ist, von der Lichtquelle 330 ausgestrahlten Gesamtlichtintensität einen im Rahmen einer Kalibrierung einstellbaren ersten Teil der Gesamtintensität aufzuteilen in den im Rahmen der Kalibrierung einstellbaren ersten Teil der Gesamtintensität, der in die erste optische Messstrecke 312a eingestellt wird und den im Rahmen der Kalibrierung einstellbaren zweiten Teil der Gesamtintensität, die in die zweite optische Messstrecke 312b eingestrahlt wird.

Dabei umfasst der Fluidbehälter zwei Bereiche bzw. Teilbehälter 310, 310 und 310', die die erste und zweite optische Messstrecke 312a und 312b definieren. Anstelle eines Fluidbehälters mit zwei Teilbereichen bzw. Teilbehältern kann auch eine Fluidleitung mit zwei Teilabschnitten vorgesehen sein, in denen die erste und zweite optische Messstrecke 312a, 312b ausgebildet ist. Der bzw. die Fluid(Teil)Behälter 310, 310' bzw. die Fluidleitung mit den zwei Teilabschnitten umfassen, wie in den Figuren 5 und 6 gezeigt, zwei planparallele, gegenüberliegende Wände, die die erste bzw. zweite optische Messstrecke 312a, 312b durch das gleichartige Fluid 302 begrenzen und den Vorteil aufweisen, die jeweilige optische Messstrecke 312a, 312b, die senkrecht zu den planparallelen Enden verläuft, besonders präzise zu definieren.

In beiden Ausführungsformen umfasst die Messeinrichtung 300 ferner einen Referenz-Lichtdetektor 370, der optisch an die Lichtquelle 330 gekoppelt ist und der dazu ausgebildet ist, ein zu der von der Lichtquelle 330 abgestrahlten Gesamtlichtintensität proportionales, elektrisches Ausgangssignal zu erzeugen, das als Referenzwert für die von der Lichtquelle 330 abgestrahlte Gesamtlichtintensität verwendet werden kann, insbesondere als Eingangsgröße in Mittel zum Regeln (Regelungseinrichtung) der von der Lichtquelle 330 abgestrahlten Gesamtlichtintensität auf ein einstellbares Niveau, entsprechend dem in Fig. 4 gezeigten Referenz-Lichtdetektor 270 der Lichtquelle 230 in der Messvorrichtung 200 gemäß dem zweiten Aspekt der Erfindung und deren oben beschriebenen Verwendung zum Regeln der abgestrahlten Gesamtlichtintensität auf ein zeitlich konstantes Level.

Die in den Figuren 5 und 6 gezeigten ersten und zweiten Ausführungsformen der Messvorrichtung 300 unterscheiden sich im Wesentlichen durch die Ausgestaltung der Lichtstrahlungsaufteilungseinrichtung 334 und die Einkopplung der von der Lichtquelle 330 ausgestrahlten ersten und zweiten Teilintensität in die erste und die zweite optische Messstrecke 312a, 312b.

In der in Fig. 5 gezeigten Ausführungsform umfasst die Lichtstrahlungsaufteilungseinrichtung 334 eine Lichtaufteilungseinrichtung 347 mit einem Lichteingang und einem ersten 349a und einem zweiten 349b Lichtausgang. Die Lichtaufteilungseinrichtung 347 ist integriert in einen sich in einen ersten Lichtleiter 340 und einen zweiten Lichtleiter 344 aufgabelnden Gesamtlichtleiter, dessen Eingang an eine Licht emittierende Einheit, wie etwa eine LED, der Lichtquelle 330 optisch gekoppelt ist. Der erste Lichtausgang 349a der Lichtaufteilungseinrichtung 347 ist gekoppelt an ein erstes Ende 341 des ersten Lichtleiters 340, dessen zweites Ende 342 an den Eingang der ersten optischen Messstrecke 312a gekoppelt ist. Entsprechend ist der zweite Lichtausgang 349b der Lichtaufteilungseinrichtung 347 gekoppelt an ein erstes Ende 345 eines zweiten Lichtleiters 344, dessen zweites Ende 346 an einem Eingang der zweiten optischen Messstrecke 312b gekoppelt ist. Dabei ist der erste bzw. zweite Lichtausgang 349a bzw. 349b jeweils mit einer Lichteintrittsfacette am jeweiligen ersten Ende 341 bzw. 345 des ersten bzw. zweiten Lichtleiters 340 bzw. 344 optisch gekoppelt. Diese Ausgestaltung der Lichtstrahlungsaufteilungseinrichtung 334 ermöglicht, von der Licht emittierenden Einheit der Lichtquelle 330 ausgesendetes Licht weitgehend abgeschirmt und verlustarm zu den räumlich weitgehend flexibel anordenbaren Positionen abgesetzt an geordneten ersten bzw. zweiten optischen Messstrecke 312a, 312b einzustrahlen.

Bei der in Fig. 6 gezeigten Ausführungsform ist die Lichtstrahlungsaufteilungseinrichtung 334 in einem offenen Strahlengangsystem angeordnet und als ein teildurchlässiger Spiegel 336 ausgebildet. Der teildurchlässige Spiegel 336 ist in einem Ausgangsstrahlengang der Licht emittierenden Einheit der Lichtquelle 330 angeordnet und teilt diesen Ausgangsstrahlengang auf in einen ersten und einen zweiten Strahlengang, in dem bzw. denen Licht zu den räumlich abgesetzt angeordneten ersten und zweiten optischen Messstrecken 312a, 312b geführt wird. Der teildurchlässige Spiegel 336 ist mit seiner planen Fläche unter einem Winkel von im Wesentlichen 45 ° in Bezug auf die Richtung des Ausgangsstrahlengangs angeordnet, reflektiert, einen ersten Teil, der zu einem reflektierenden Spiegel 337 führt und von dort in die Richtung zu und zum Eingang der ersten optischen Messstrecke 312a reflektiert wird. Der teildurchlässige Spiegel 336 lässt einen zweiten Teil des Ausgangsstrahls der Licht emittierenden Einheit der Lichtquelle 330 durch, so dass dieser zum Eingang der zweiten optischen Messstrecke 312b führt. Es empfiehlt sich, das in Fig. 6 gezeigte, offen geführte Strahlengangsystem als Ganzes in einem Innengehäuse (nicht gezeigt) einzukapseln und vom Umgebungslicht abzuschirmen.

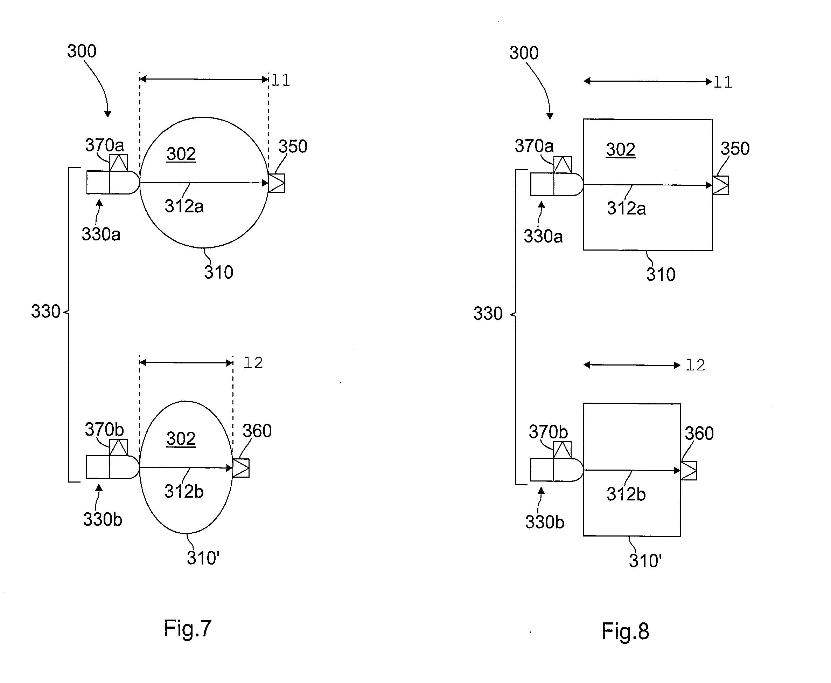

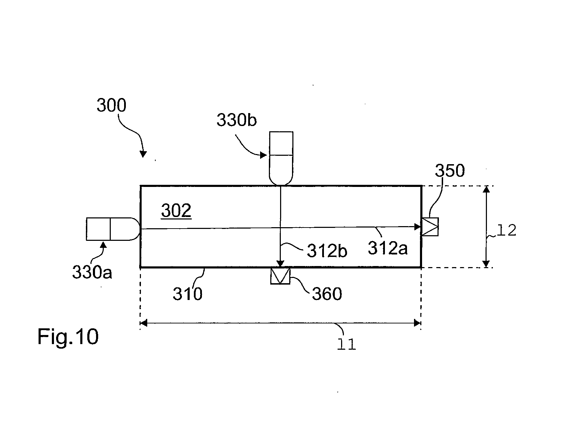

Die in den Figuren 7 und 8 schematisch gezeigte dritte und vierte Ausführungsformen der Messvorrichtung 300 gemäß dem ersten Aspekt der Erfindung unterscheiden sich von den in den in den Figuren 5 und 6 gezeigten ersten und zweiten Ausführungsformen im Wesentlichen durch die Art bzw. die verwendeten Mittel zum Erzeugen bzw. Aufteilen bzw. Einstrahlen des ersten und zweiten Licht-Intensitätsanteils aus der Lichtquelle 330 (Gesamtlichtquelle) in die erste und zweite optische Messstrecke 312a, 312b, und die in Fig. 7 gezeigte Ausführungsform ferner noch durch die Ausgestaltung des Fluidbehälters für das Fluid 302 mit den Teil-Fluidbehältern 310, 310'.

Bei den in den Figuren 7 und 8 gezeigten Ausführungsformen umfasst die Lichtquelle 330 (Gesamtlichtquelle) eine erste und eine zweite Teil-Lichtquelle 330a, 330b, die einen jeweiligen ersten Intensitätsanteil in die erste bzw. zweite optische Messstrecke 312a, 312b einstrahlen. Dabei sind die erste und die zweite Teil-Lichtquelle 330a, 330b jeweils als eigenständig hinsichtlich ihrer Ausstrahlungsintensität ansteuerbare Licht emittierende Einheiten, wie etwa eine LED, die direkt an einen Eingang der jeweiligen optischen Messstrecke 312a, 312b optisch gekoppelt ist, ausgebildet. In dieser Ausführungsform der Lichtquelle 330 wird im Vergleich zu der Fig. 5 der sich in den ersten und zweiten Lichtleiter 340, 344 aufgabelnden Gesamtlichtleiter und im Vergleich zur Fig. 6 das sich aufgabelnde optische Strahlungswegsystem eingespart. Die erste und zweite Teil-Lichtquelle 330a, 330b umfasst einen jeweiligen ersten und zweiten Referenz-Lichtdetektor 370a, 370b, der direkt an die Licht emittierende Einheit der jeweiligen Teil-Lichtquelle 330a, 330b optisch gekoppelt ist. Der erste bzw. zweite Referenz-Lichtdetektor 370a, 370b ist dazu ausgebildet, ein von der Licht emittierenden Einheit abgestrahlten Lichtintensität proportionales, elektrisches Ausgangssignal zu erzeugen, das als Referenzwert für die von der Teil-Lichtquelle 330a, 330b abgestrahlten Teil-Lichtintensität verwendet werden kann und, wie bereits beschrieben, zum Regeln der abgestrahlten Lichtintensität auf ein zeitlich konstantes Niveau verwendet werden kann.

In jeweiligen Varianten (nicht gezeigt) der in den Figuren 7 und 8 gezeigten Ausführungsformen sind die erste und zweite Teil-Lichtquelle 330a, 330b ersetzt durch ein zweites Ende (entsprechend den in Fig. 5 gezeigten zweiten Enden 342, 346) von Lichtleitern (entsprechend den in Fig. 5 gezeigten ersten und zweiten Lichtleitern 340, 344). Dabei erfolgt die Lichterzeugung und Aufteilung mittels einer (Gesamt-) Lichtquelle 330 und einer Lichtstrahlungsaufteilungseinrichtung 334 entsprechend zum Beispiel der in Fig. 5 gezeigten Ausführungsform.

Die in der Figur 9 schematisch gezeigte fünfte Ausführungsform der Messvorrichtung 300 gemäß dem ersten Aspekt der Erfindung unterscheidet sich von den in den Figuren 5 bis 8 gezeigten Ausführungsformen im Wesentlichen durch die Ausgestaltung des Fluidbehälters 310 bzw. Fluidleitung mit dem darin enthaltenen Fluid 302. In dem in Fig. 9 gezeigten einen Fluidbehälter 310 bzw. der Fluidleitung sind die erste und die zweite optische Messstrecke 312a und 312b in einem Bereich des Fluidbehälters 310 bzw. der Fluidleitung ausgebildet, welcher Bereich einen rechteckförmigen Querschnitt aufweist, der die erste und zweite optische Messstrecke 312a und 312b enthält, wie in Fig. 9gezeigt. Der rechteckförmige Querschnitt des Fluidbehälters 310 bzw. der Fluidleitung ist begrenzt bzw. definiert durch ein erstes Paar von planparallel zueinander angeordneten Wänden (in Fig. 9 die kurzen Wände des rechteckförmigen Querschnitts, nicht bezeichnet), die in einem Abstand zueinander angeordnet sind, der der Länge L₁ der ersten optischen Messstrecke 312a entspricht, und zwischen denen die erste optische Messstrecke 312a definiert ist, wie in Fig. 9 gezeigt. Der Fluidbehälter 310 bzw. die Fluidleitung umfasst ferner ein zweites Paar planparallel zueinander angeordnete Wände (in Fig. 9 die langen Wände des rechteckigen Querschnitts, nicht bezeichnet), die in einem Abstand zueinander angeordnet sind, der der Länge L₂ der zweiten optischen Messstrecke 312b entspricht, und zwischen denen die optische Messstrecke 312b definiert ist. Ein erster Licht-Intensitätsanteil wird in die erste optische Messstrecke 312a eingestrahlt mittels zum Beispiel einer eigenständig ansteuerbaren ersten Teil-Lichtquelle 330a (entsprechend den in den Figuren 7 und 8 gezeigten Ausführungsformen) oder ein zweites Ende (nicht gezeigt) oder ein zweites Ende (entsprechend 342 in Fig. 5) eines ersten Lichtleiters (entsprechend 340 in der in Fig. 5 gezeigten Ausführungsform). Ein zweiter Licht-Intensitätsanteil wird in die zweite optische Messstrecke 312b eingestrahlt mittels entweder einer eigenständig ansteuerbaren zweiten Teil-Lichtquelle 330b (wie in den Figuren 7 und 8 gezeigt).

Im Betrieb bzw. der Inbetriebnahme der Messvorrichtung 300 wird gemäß dem Verfahren gemäß dem dritten Aspekt der Erfindung Licht mit einem ersten Intensitätsanteil in die erste optische Messstrecke 312a und Licht mit einem zweiten Intensitätsanteil in die zweite optische Messstrecke 312b mittels der Lichtquelle 300 eingestrahlt, wie beispielsweise in den Figuren 5 bis 9 gezeigt. Dann wird mittels des ersten und des zweiten Lichtdetektors 350 und 360 eine erste und eine zweite Intensität von Licht gemessen, das von der Lichtquelle 330 ausgesendet worden ist und die erste bzw. zweite optische Messstrecke 312a bzw. 312b durch Laufen hat. Im Rahmen einer Kalibration wird dann die von der Lichtquelle 330 abgestrahlte Gesamtlichtintensität (vgl. die Figuren 5 und 6) bzw. eine von der ersten Teil-Lichtquelle 330a abgestrahlte erste Teil-Lichtintensität so eingestellt, dass sich für eine erste, insbesondere relativ niedrige bis vernachlässigbare (d. h. null) Konzentration der Substanz in dem Fluid 302 die nach Durchlaufen der voreingestellten Länge L_{1'} der ersten optischen Messstrecke 312a an dem ersten Lichtdetektor 350 eintreffende erste Lichtintensität in dessen ersten Messbereich fällt. Das heißt, ein erster Messkanal umfassend die erste optische Messstrecke 312a und deren ersten Lichtdetektor 350 wird für eine Messung der geringen Konzentration der Substanz in dem Fluid 302 für eine Messung bei optimaler Auflösung kalibriert bzw. eingestellt. Nach dieser Kalibration kann mittels des ersten Lichtdetektors 350 die erste Intensität gemessen bzw. ihr zeitlicher Verlauf erfasst werden als Maß für die optische Absorbanz des entlang der ersten optischen Messstrecke 312a vorhandenen Fluids 302. Gleichzeitig dazu kann in einem zweiten Messkanal umfassend die zweite optische Messstrecke 312b und den zweiten Lichtdetektor 360 eine zweite Intensität gemessen bzw. ihr zeitlicher Verlauf erfasst werden von Licht, das die zweite optische Messstrecke 312b durchlaufen hat, als zweites Maß für die optische Absorbanz des entlang der zweiten optischen Messstrecke 312b vorhandenen gleichartigen Fluids 302. Weil nun die zweite optische Messstrecke 312b kürzer, insbesondere signifikant kürzer als die erste optische Messstrecke 312a ist, ist der zweite Messkanal besser dazu geeignet, optische Absorbanzen hervorgerufen durch eine relativ hohe Konzentration der Substanz in dem Fluid 302 zu messen, und zwar mit nahezu bzw. optimaler Auflösung zu messen, dies im Gegensatz zum ersten Messkanal, in dem bei hoher Konzentration der Substanz in dem Fluid 302 aufgrund der relativ großen Länge der ersten optischen Messstrecke 312a auf der ersten optischen Messstrecke 312a relativ viel Licht absorbiert bzw. gestreut wird und nur eine sehr geringe Lichtintensität den ersten Lichtdetektor 350 erreicht, so dass der erste Lichtdetektor 350 dann in einem (unteren bzw. niedrigen) Intensitätsmessbereich, der ggf. auch außerhalb bzw. unterhalb des ersten Messbereichs des Lichtdetektors 350 fällt. Auf diese Weise ist die Messvorrichtung 300 gemäß dem ersten Aspekt der Erfindung aufgrund der ersten und zweiten vorgesehenen optischen Messstrecke 312a und 312b mit unterschiedlichen Längen besonders gut dazu geeignet, optische Absorbanzen von dem Fluid 302 gelösten bzw. mitgeführten Substanzen sowohl bei relativ (sehr) geringer Konzentration als auch bei relativ (sehr) hoher Konzentration der Substanz mit jeweils optimaler Auflösung in dem ersten bzw. zweiten Messkanal zu messen.

### Bezugszeichenliste:

- 10: Patient
- 20: extrakorporaler Blutkreislauf
- 22: arterieller Zugang
- 24: arterielles Schlauchsystem
- 26: arterieller Luftfänger
- 28: erster Drucksensor (arterienseitiger Druck)
- 30: arterielle Blutpumpe
- 32: zweiter Drucksensor (Blutbereichseingangsdruck)
- 34: Bluteinlass
- 36: Blutauslass
- 38: Ausgleichsbehälter
- 42: dritter Drucksensor (venöser Rückstaudruck)
- 44: venöses Schlauchsystem
- 46: venöser Luftfänger
- 48: venöser Zugang
- 50: Dialysatoreinrichtung
- 52: Blutbereich
- 54: Dialysatbereich
- 56: Membranfiltereinrichtung
- 60: Dialysatkreislauf
- 62: Dialysataufbereitung
- 64: Flusspumpe im Dialysatzufluss
- 66: erstes Bypassventil
- 68: Bypass-Leitung
- 70: zweites Bypassventil
- 72: Dialysatzufuhrleitung
- 74: Dialysateinlass
- 76: Dialysatauslass
- 78: Dialysatabfuhrleitung
- 80: Konzentrationsmesseinrichtung, z.B. UV-Messeinrichtung
- 82: Dialysatabfluss
- 100: Dialysemaschine
- 102: Maschinengehäuse
- 110: Ein-/Ausgabeeinrichtung
- 120: Steuer- und Regelungseinheit
- 200: fotometrische Messvorrichtung
- 202: Fluid
- 210: Fluidbehälter
- 211: Umfangswandungsabschnitt
- 212: optische Messstrecke
- 214: erste/r Wand bzw. Wandungsabschnitt
- 215: zweite/r Wand bzw. Wandungsabschnitt
- 218: erste Begrenzungsbacke
- 219: zweite Begrenzungsbacke
- 230: Lichtquelle
- 250: Lichtdetektor
- 270: Referenz-Lichtdetektor
- 300: fotometrische Messvorrichtung
- 302: Fluid
- 310: Fluidbehälter, Teil-Fluidbehälter
- 310': Teil-Fluidbehälter
- 312a: erste optische Messstrecke
- 312b: zweite optische Messstrecke
- 330: Lichtquelle
- 330a: erste Teil-Lichtquelle
- 330b: zweite Teil-Lichtquelle
- 334: Lichtstrahlungsaufteilungseinrichtung
- 336: teildurchlässiger Spiegel
- 337: reflektierender Spiegel
- 340: erster Lichtleiter
- 341: erstes Ende
- 342: zweites Ende
- 344: zweiter Lichtleiter
- 345: erstes Ende
- 346: zweites Ende
- 347: Lichtaufteilungseinrichtung
- 348: Lichteingang
- 349a: erster Lichtausgang
- 349b: zweiter Lichtausgang
- 350: erster Lichtdetektor
- 360: zweiter Lichtdetektor
- 370: Referenz-Lichtdetektor
- 370a: erster Referenz-Lichtdetektor
- 370b: zweiter Referenz-Lichtdetektor

## Patentansprüche

1. Fotometrische Messvorrichtung (300) zum Messen einer optischen Absorbanz eines Fluids (302), wie etwa eines in einem Dialysatabfluss (78) einer Dialysemaschine (100) strömenden Dialysats, wobei die Messvorrichtung (300) umfasst:
mindestens eine erste und eine zweite durch das Fluid (302) verlaufende optische Messstrecke (312a, 312b),
eine Lichtquelle (330), die dazu ausgebildet ist, Licht mit einem ersten Intensitätsanteil in eine durch das Fluid (302) verlaufende erste optische Messstrecke (312a) einzustrahlen und Licht mit einem zweiten Intensitätsanteil in eine zweite, durch das Fluid (302) verlaufende optische Messstrecke (312b) einzustrahlen,
mindestens einen ersten und einen zweiten Lichtdetektor (350, 360),
wobei der erste Lichtdetektor (350) dazu ausgebildet ist, eine erste Intensität von auf dem ersten Lichtdetektor (350) auftreffendem Licht, das von der Lichtquelle (330) ausgesendet worden ist und die erste optische Messstrecke (312a) durchlaufen hat, zu messen und ein erstes elektrisches Ausgangssignal zu erzeugen,
wobei der zweite Lichtdetektor (360) dazu ausgebildet ist, eine zweite Intensität von auf dem zweiten Lichtdetektor (360) auftreffendem Licht, das von der Lichtquelle (330) ausgesendet worden ist und die zweite optische Messstrecke (312b) durchlaufen hat, zu messen und ein zweites elektrisches Ausgangssignal zu erzeugen, und
einen Referenz-Lichtdetektor (270; 370, 370a, 370b), der optisch an die Lichtquelle (230; 330, 330a, 330b) gekoppelt ist und der dazu ausgebildet ist, ein elektrisches Ausgangssignal zu erzeugen, das in Beziehung zu der von der Lichtquelle (230; 330, 330a, 330b) abgestrahlten Gesamtlichtintensität steht und das als Referenzwert für die von Lichtquelle (230; 330, 330a, 330b) abgestrahlte Gesamtlichtintensität verwendbar ist.

2. Messvorrichtung (300) nach Anspruch 1, die dazu ausgelegt ist, dass im Betrieb der erste Lichtdetektor (350) in einem ersten Intensitätsmessbereich und gleichzeitig der zweite Lichtdetektor (360) in einem zweiten Intensitätsmessbereich betrieben werden kann.

3. Messvorrichtung (300) nach Anspruch 1 oder 2, die so ausgelegt ist, dass im Betrieb die erste optische Messstrecke (312a) zusammen mit dem ersten Lichtdetektor (350) zum Messen einer ersten, insbesondere relativ niedrigen Konzentration der Substanz in dem Fluid (302) ausgelegt ist, und dass die zweite optische Messstrecke (312b) zusammen mit dem zweiten Lichtdetektor (360) zum Messen einer zweiten, insbesondere relativ hohen Konzentration der Substanz in dem Fluid (302) ausgelegt ist, wobei die zweite Konzentration größer als die erste Konzentration ist.

4. Messvorrichtung (300) nach einem der Ansprüche 1 bis 3, bei der die Lichtquelle (330) eine Lichtstrahlungsaufteilungseinrichtung (334) umfasst, die dazu ausgebildet ist, einen, optional einstellbaren, ersten Teil der Gesamtintensität in die erste optische Messstrecke (312a) einzustrahlen und einen, optional einstellbaren, zweiten Teil der Gesamtintensität in die zweite optische Messstrecke (312b) einzustrahlen.

5. Messvorrichtung nach Anspruch 4, bei der die Lichtstrahlungsaufteilungseinrichtung (334) folgendes umfasst:
einen ersten Lichtleiter (340), wie etwa eine Lichtleitfaser, mit einem ersten und einem zweiten Ende (341, 342), wobei das zweite Ende (342) an einen Anfang der ersten optischen Messstrecke (312a) optisch ankoppelbar ist,
einen zweiten Lichtleiter (344), wie etwa eine Lichtleitfaser, mit einem ersten und einem zweiten Ende (345, 346), wobei das zweite Ende (346) an einen Anfang der zweiten optischen Messstrecke (312b) optisch ankoppelbar ist, und
eine Lichtaufteilungseinrichtung (347) mit einem Lichteingang (348) sowie einem ersten und einem zweiten Lichtausgang (349a, 349b), die dazu ausgebildet ist, in den Lichteingang (348) eingestrahltes Licht aufzuteilen in einen durch den ersten Lichtausgang (349a) austretenden ersten Teil und einen durch den zweiten Lichtausgang (349b) austretenden zweiten Teil, wobei zumindest ein Teil der von der Lichtquelle (330) ausgestrahlten Gesamtintensität durch den Lichteingang (348) in die Lichtaufteilungseinrichtung (347) einkoppelbar ist, der erste Lichtausgang (349a) an das erste Ende (341) des ersten Lichtleiters (340) optisch ankoppelbar ist und der zweite Lichtausgang (349b) an das erste Ende (345) des zweiten Lichtleiters (344) ankoppelbar ist, und
wobei insbesondere der erste Lichtausgang (349a) der Lichtaufteilungseinrichtung (347) mit einer Lichteintrittsfacette des ersten Lichtleiters (340) und der zweite Lichtausgang (349b) der Lichtaufteilungseinrichtung (347) mit einer Lichteintrittsfacette des zweiten Lichtleiters (344) optisch gekoppelt ist.

6. Messvorrichtung nach einem der Ansprüche 4 und 5, bei der der erste Lichtdetektor (350) an ein Ende der ersten optischen Messstrecke (312a) optisch ankoppelbar ist und insbesondere eine erste Licht detektierende Einheit, wie etwa eine Photodiode, umfasst, und dass der zweite Lichtdetektor (360) an ein Ende der zweiten optischen Messstrecke (312b) optisch ankoppelbar ist und insbesondere eine zweite Licht detektierende Einheit, wie etwa eine Photodiode, umfasst.

7. Fotometrische Messvorrichtung (200) zum Messen einer optischen Absorbanz eines Fluids (202), wie etwa eines in einem Dialysatabfluss (78) einer Dialysemaschine (100) strömenden Dialysats, wobei die Messvorrichtung (200) folgendes umfasst:
eine Lichtquelle (230), die dazu ausgebildet ist, Licht in eine optische Messstrecke (212) einzustrahlen, und
mindestens einen Lichtdetektor (250), der dazu ausgebildet ist, eine Intensität von auf dem Lichtdetektor (250) auftreffendem Licht, das von der Lichtquelle (230) ausgesendet worden ist und eine optische Messstrecke (212) durchlaufen hat, zu messen und ein elektrisches Ausgangssignal zu erzeugen,
einen Referenz-Lichtdetektor (270; 370, 370a, 370b), der optisch an die Lichtquelle (230; 330, 330a, 330b) gekoppelt ist und der dazu ausgebildet ist, ein elektrisches Ausgangssignal zu erzeugen, das in Beziehung zu der von der Lichtquelle (230; 330, 330a, 330b) abgestrahlten Gesamtlichtintensität steht und das als Referenzwert für die von Lichtquelle (230; 330, 330a, 330b) abgestrahlte Gesamtlichtintensität verwendbar ist,
wobei die Messvorrichtung (200) dazu ausgebildet ist, dass im Betrieb für eine vorbestimmte, von der Lichtquelle (230) ausgesendete Lichtintensität und eine zu erwartende Konzentration der Substanz in dem Fluid (202) die Länge (I₁, I₂, I₃) der mindestens einen optischen Messstrecke (212) durch das Fluid (202) so einstellbar ist, dass die an dem Lichtdetektor (250) eintreffende Lichtintensität in dessen Intensitätsmessbereich fällt.

8. Messvorrichtung nach Anspruch 7, mit einem Fluidbehälter (210) und/oder einer Fluidleitung, die Mittel (214, 215, 218, 219) zum Einstellen der Länge (I₁, I₂, I₃) der optischen Messstrecke (212) umfassen.

9. Messvorrichtung (200; 300) nach einem der Ansprüche 1 bis 8, wobei das elektrische Ausgangssignal proportional zu der von der Lichtquelle (230; 330, 330a, 330b) abgestrahlten Gesamtlichtintensität ist.

10. Messvorrichtung (200; 300) nach einem der Ansprüche 1 bis 9, mit Mitteln zum Regeln der von der Lichtquelle (230; 330, 330a, 330b) abgestrahlten Gesamtlichtintensität auf ein einstellbares Niveau unter Benutzung des Ausgangssignals des Referenz-Lichtdetektors (270; 370, 370a, 370b).

11. Messvorrichtung (200; 300) nach einem der Ansprüche 1 bis 10, mit mindestens einem mechanischen Mittel zum Einstellen einer vorbestimmten Solllänge der einstellbare Länge aufweisenden optischen Messstrecke (212) bzw. der ersten optischen Messstrecke (312a) und/oder der zweiten optischen Messstrecke (312b).

12. Blutbehandlungsvorrichtung, insbesondere Dialysemaschine, mit einer fotometrischen Messvorrichtung (200, 300) nach einem der Ansprüche 1 bis 11.

13. Verfahren zum Messen einer optischen Absorbanz eines Fluids (302), wie etwa eines in einem Dialysatabfluss (78) einer Dialysemaschine (100) strömenden Dialysats, mit den Schritten
Einstrahlen von Licht mit einem ersten Intensitätsanteil in die erste optische Messstrecke (312a) und von Licht mit einem zweiten Intensitätsanteil in die zweite optische Messstrecke (312b) mittels einer Lichtquelle (330),
Messen einer ersten Intensität von Licht, das von der Lichtquelle (330) ausgesendet worden ist und die erste optische Messstrecke (312a) durchlaufen hat, mittels eines ersten Lichtdetektors (350), der dazu ausgebildet ist, ein erstes elektrisches Ausgangssignal zu erzeugen, und
Messen, mittels des ersten Lichtdetektors (350), der ersten Intensität bzw. Erfassen eines zeitlichen Verlaufs der ersten Intensität von Licht, das von der Lichtquelle (330) ausgesendet worden ist und die erste optische Messstrecke (312a) durchlaufen hat, als Maß für die optische Absorbanz des entlang der ersten optischen Messstrecke (312a) vorhandenen Fluids (302), und, vorzugsweise gleichzeitig dazu,
Messen, mittels eines zweiten Lichtdetektors (360), der zweiten Intensität bzw. Erfassen eines zeitlichen Verlaufs einer zweiten Intensität von Licht, das von der Lichtquelle (330) ausgesendet worden ist und die zweite optische Messstrecke (312b) durchlaufen hat, als Maß für die optische Absorbanz des entlang der zweiten optischen Messstrecke (312b) vorhandenen gleichartigen Fluids (302), und
Erzeugen, mittels eines Referenz-Lichtdetektors (270; 370, 370a, 370b), der optisch an die Lichtquelle (230; 330, 330a, 330b) gekoppelt ist, eines elektrischen Ausgangssignals, das in Beziehung zu der von der Lichtquelle (230; 330, 330a, 330b) abgestrahlten Gesamtlichtintensität steht und das als Referenzwert für die von Lichtquelle (230; 330, 330a, 330b) abgestrahlte Gesamtlichtintensität verwendbar ist.

14. Verfahren zum Messen einer optischen Absorbanz eines Fluids (202), wie etwa eines in einem Dialysatabfluss (78) einer Dialysemaschine (100) strömenden Dialysats, mit den Schritten:
Einstrahlen von Licht mit einem ersten Intensitätsanteil in eine optische Messstrecke (212) mittels einer Lichtquelle (230),
Messen einer ersten Intensität von Licht, das von der Lichtquelle (230) ausgesendet worden ist und die optische Messstrecke (212) mit einer ersten Länge (I₁) durchlaufen hat, mittels eines Lichtdetektors (250), der dazu ausgebildet ist, ein elektrisches Ausgangssignal zu erzeugen,
Messen der Intensität bzw. Erfassen eines zeitlichen Verlaufs der Intensität von auf dem Lichtdetektor (250) auftreffendem Licht als Maß für die Absorbanz des in der optischen Messstrecke (212) vorhandenen Fluids (202),
Neu-Einstellen der Länge der optischen Messstrecke, wenn die an dem Lichtdetektor (250) eintreffende Lichtintensität nicht mehr in dessen Messbereich fällt, derart, dass die an dem Lichtdetektor (250) eintreffende Lichtintensität wieder in dessen Messbereich fällt,
Messen der Intensität bzw. Erfassen eines zeitlichen Verlaufs der Intensität von auf den Lichtdetektor (250) auftreffendem Licht als Maß für die Absorbanz des entlang der optischen Messstrecke (212) vorhandenen Fluids (202) mit der neu-eingestellten Länge (I₂), und
Erzeugen, mittels eines Referenz-Lichtdetektors (270; 370, 370a, 370b), der optisch an die Lichtquelle (230; 330, 330a, 330b) gekoppelt ist, eines elektrischen Ausgangssignals, das in Beziehung zu der von der Lichtquelle (230; 330, 330a, 330b) abgestrahlten Gesamtlichtintensität steht und das als Referenzwert für die von Lichtquelle (230; 330, 330a, 330b) abgestrahlte Gesamtlichtintensität verwendbar ist..

15. Verfahren nach Anspruch 14, ferner umfassend:
Wiederholen der Schritte des Neu-Einstellens und des Messens der Intensität mit der neu-eingestellten Länge während einer einstellbaren Messdauer zum Erfassen eines zeitlichen Verlaufs der optischen Absorbanz des entlang der optischen Messstrecke (212) vorhandenen Fluids (202) mit Berücksichtigung der jeweils eingestellten Länge der optischen Messstrecke (212).
